# EUROPÄISCHE PATENTANMELDUNG

(11) **EP 1 088 585 A2**
(43) Veröffentlichungstag der Anmeldung: **04.04.2001**
(21) Anmeldenummer: 00117339.2
(22) Anmeldetag: 17.02.1998
(51) Int. Cl.: B01J 19/00, C07B 61/00

(54) **Automatisiertes simultanes chemisches Syntheseverfahren**

(30) Priorität: 17.02.1997 DE 19706089
(62) Teilanmeldung aus: 98913553.8
(71) Anmelder: Gesellschaft für Biotechnologische Forschung mbH (GBF), 38124 Braunschweig (DE); ABIMED Analysen-Technik Gesellschaft mit beschränkter Haftung, 40736 Langenfeld (DE); IMB Institut fur Molekulare Biotechnologie E.V., 07745 Jena (DE)
(72) Erfinder: Die Erfindernennung liegt noch nicht vor
(74) Vertreter: Boeters, Hans Dietrich, Dr.

(57) **Zusammenfassung**

Die Erfindung betrifft ein automatisiertes simultanes chemisches Syntheseverfahren, das dadurch gekennzeichnet ist, daß für die kovalente Verknüpfung der Produkte mit Trägermaterial geeignete Verknüpfungsbausteine bzw. Linker vorgesehen werden, die eine selektive und schonende finale Abspaltung der Produkte erlauben, wobei insbesondere für die Synthese von oligomeren Verbindungen, insbesondere von Oligonucleotiden oder Peptiden, ein universeller Linker vorgesehen wird, an den auch die Bausteine der ersten Aufbaureaktion mit dem gleichen chemischen Reaktionstyp verknüpft werden können, der auch für die weiteren Aufbaureaktionen eingesetzt wird, wodurch für die ganze Synthese einer Verbindungsklasse nur ein Typ von Bausteinen gebraucht wird, insbesondere nur ein Nucleosid-3'-phosphoramidit für die Synthese von 3'-OH-Oligonucleotiden.

## Beschreibung

Die Synthese von polymeren Biomolekülen, wie Oligonucleotide, Peptide oder unnatürliche Analoge dazu, nach dem Prinzip der Festphasensynthese ist eine etablierte Technik (2).

Während mutiple parallele Peptidsynthese in offenen Reaktionssystemen zum Stand der Technik gehört (14, 15), werden z.B. Oligonucleotide eher einzeln hergestellt. Das Hauptproblem der Oligonucleotidsynthese liegt in der extremen Wasserempfindlichkeit der zum Stand der Technik gehörenden Phosphoramiditchemie (2). Oligosyntheseaustomaten sind daher geschlossene Systeme und arbeiten unter Schutzgas. Ein plubiziertes Gerät zur parallelen Synthese von bis zu 96 Oligonucleotiden benutzt ein ebenfalls geschlossenes Reaktionssystem und eine Vielzahl von Ventilen zur Dosierung von Reagenzien (16). Hier werden konventionelle Träger und manuelle Prozeduren zur Aufarbeitung verwendet.

Dem erfindungsgemäßen Synthesesystem liegt nun der Gedanke zugrunde, Synthese, Träger, Ankergruppen und Aufarbeitungsprozedur für die simultane vollautomatische Herstellung von Biomolekülen auszulegen. Bei Verteilung der Reagenzien über einen Pipettierroboter können die Reaktionssäulen in einem für die weitere Bearbeitung geeigneten Format angeordnet werden. Um mit einem Pipettierroboter auch ein wasser- und luftempfindliches Syntheseprotokoll durchführen zu können, müssen bestimmte konstruktive Maßnahmen ergriffen werden. Beispielhaft für eine mögliche Lösung werden im folgenden das Funktionsprinzip des Automaten und der Ablauf einer Synthese beschrieben.

Der Automat kann mit konventionellen Trägern und Reagenzien arbeiten. Die Handhabung wird aber durch speziell angepaßte, neu entwickelte Träger und Ankergruppen vereinfacht.

Durch Anschluß eines speziellen Verfahrens für simultane Reinigung und Aliquotierung wird die Qualität der Produkte verbessert und die Verwendung vereinfacht.

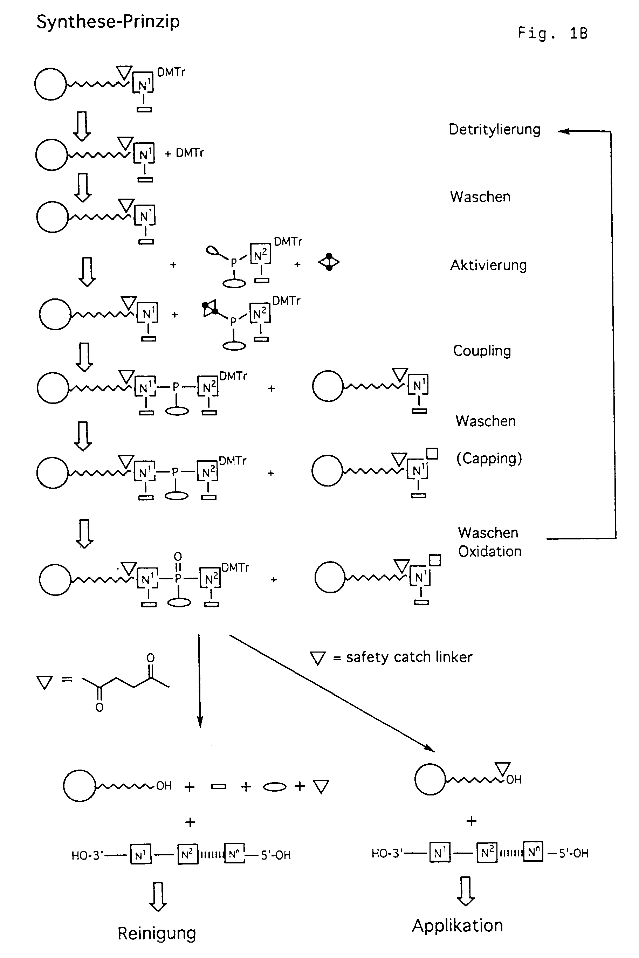

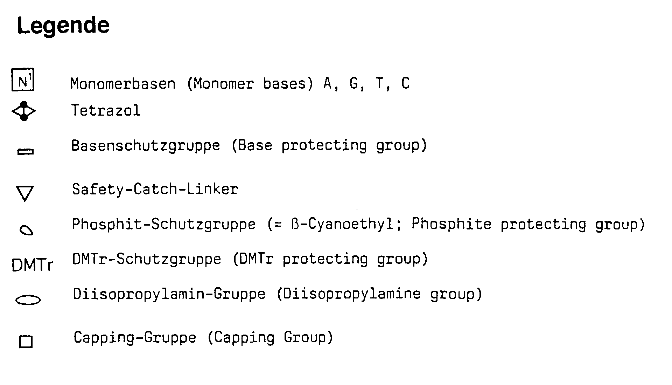

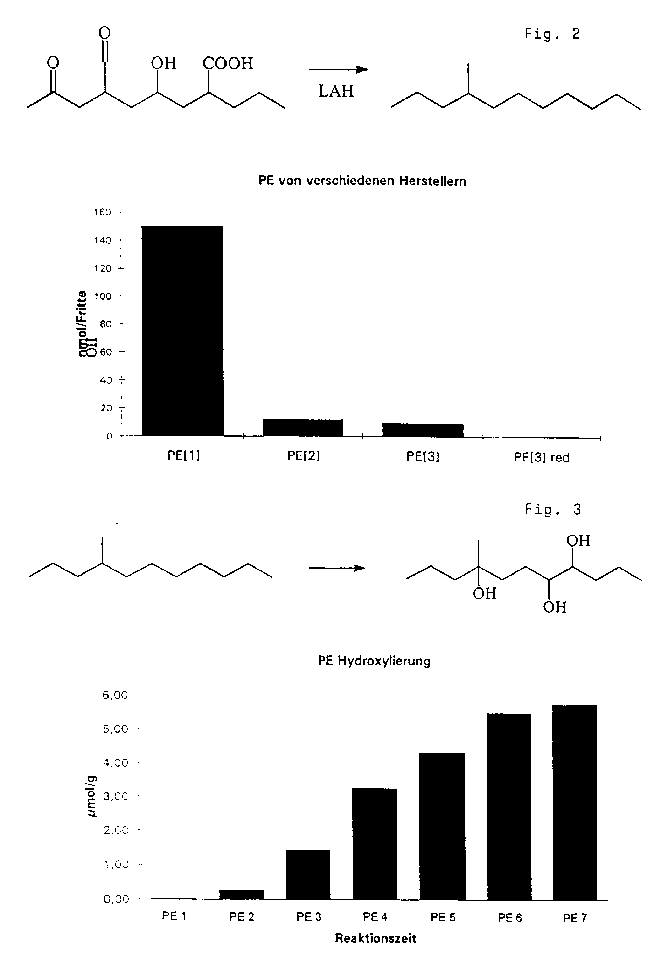

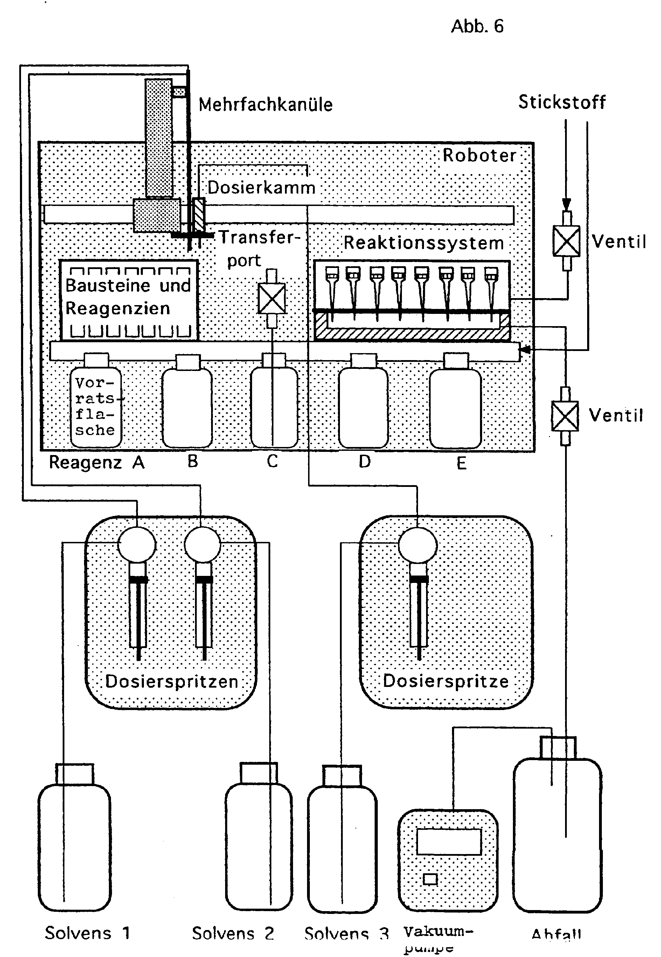

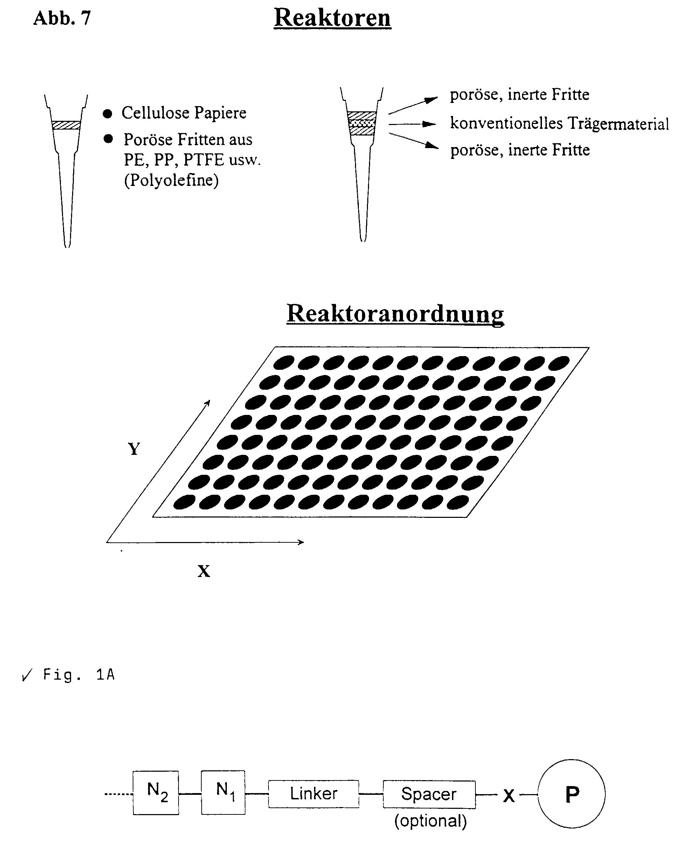

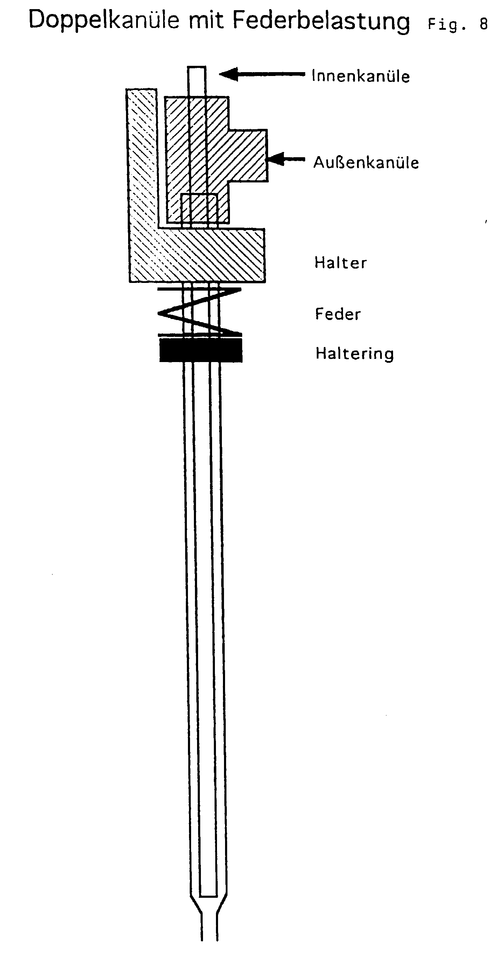

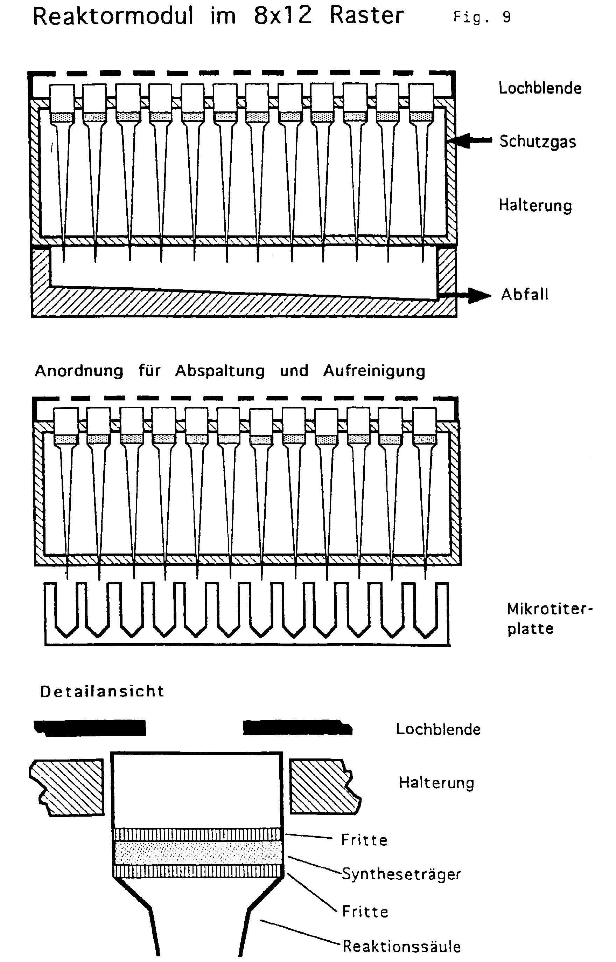

Insbesondere betrifft die Erfindung folgende Ausführungsformen: Vorrichtung für eine automatisierte simultane chemische Synthese und Aufreinigung einer Vielzahl von Produkten an der Festphase sowie Trägermaterial und chemische Bausteine für die Festphasensynthese, dadurch gekennzeichnet, daß
1. eine Vielzahl (10 bis 1000, vorzugsweise 48 und ein Vielfaches davon, vorzugsweise 400) von separaten, nach oben und unten offenen Reaktionsgefäßen als parallel in einem Block (**Abb. 6**) angeordnete Kanäle oder kleine Säulen vorgesehen werden, die entweder gemeinsam oder einzeln entnehmbar sind; in den Kanälen/Säulen das Trägermaterial für die Synthese (Festphase) entweder zwischen zwei inerte poröse Frittenböden oder vorzugsweise selbst als chemisch modifizierter Fritten- oder Filterboden eingebracht wird **(Abb. 7**), so daß von oben zugegebene flüssige Medien allein durch die Oberflächenspannung und Benetzung des Materials im Reaktor festgehalten werden;
2. der Reaktor Reaktorblock nach 1. auf eine Wanne aufgesetzt wird, die über ein schaltbares Ventil an eine Vakuumpumpe angeschlossen ist und so die flüssigen Medien aus den Reaktoren und den darin enthaltenen Trägermaterialien simultan abgesaugt werden können;
3. die oberen Eingänge der Reaktionssäulen im Reaktorblock nach 1. abgedeckt durch eine darüber angebrachte Lochblende (Prallblech) mit Inertgas (z.B. Stickstoff, Argon) geflutet werden können und der Inertgasstrom gegebenenfalls während des Absaugvorganges nach 2. deutlich erhöht wird; alternativ, der Raum über den Reaktionssäulen/-kanälen durch eine zweite, verschiebbare Lochblende gezielt verschlossen werden kann, um die Reagenzien mit Inertgasüberdruck aus den Reaktionssäulen/-kanälen auszublasen;
4. chemische Bausteine, Reagenzien und Lösungsmittel von einem xyz-Pipettierroboter über elektronisch steuerbare Dosierspritzen (Dilutoren) mit einer oder mehreren Dosiernadeln und gegebenenfalls zusätzlich einem oder mehreren Dosierkämmen auf die Reaktionsgefäße verteilt werden, so daß jeder Reaktor einzeln addressierbar ist;
5. die Dosiernadel nach 4. mit mehreren (mindestens zwei) an separate Dosierspritzen angeschlossenen, also getrennt befüllbaren inneren Kanälen ausgerüstet ist, deren Enden sich erst durch vor dem Auslaß treffen (**Abb. 8**) und so bei simultaner Dosierung mehrerer Reagenzien die Mischung erst kurz vor der Abgabe in der Spitze der Dosiernadel erfolgt, wobei ein Kanal auch an die Inertgasversorgung angeschlossen sein kann und so über ein Inertgaspuls das Mischvolumen ausgedrückt werden kann;
6. die Dosiernadel nach 4. und 5. in der Längsachse federnd gelagert ist, um ohne Beschädigung auf das Trägermaterial oder die Deckfritten in den Reaktorkanälen aufsetzen zu können und so auch kleinste Volumina bis heruntr zu 1 Nanoliter sicher absetzen zu können;
7. eine Vielzahl (zwei bis einhundert, vorzugsweise 24) an chemischen Bausteinen und Reagenzien, gegebenenfalls in geeigneten Lösungsmitteln gelöst, in mit Septen verschlossenen Gefäßen, die in einem vom Reaktionsblock getrennten Reagenzienblock angeordnet sind, bereitgestellt werden;
8. die mit Septen verschlossenen Hälse der Gefäße im Reaktorblock nach 7. abgedeckt durch eine darüber angebrachte Lochblende (Prallblech) mit Inertgas (z.B. Stickstoff, Argon) geflutet werden können;
9. Reagenzien mit der Dosiernadel nach 4. und 5. auch aus Transferports entnommen werden können, die entweder direkt oder über schaltbare Ventile mit Vorratsflaschen verbunden sind (**Abb. 6**) und diese Vorratsflaschen unter einem geringen Überdruck an Inertgas stehen;
10. Lösungsmittel und Reagenzien aus Solventflaschen mittels Dosierspritzen oder durch Inertgasüberdruck auch über einen oder mehrere Dosierkämme nach 4. simultan auf mehrere Reaktoren reihenweise verteilt werden können;
11. das Trägermaterial nach 1. eine Schicht im Reaktorkanal bildet, die gleichmäßig von den oben aufgegebenen Reagenzien und Lösungsmitteln allein durch die Schwerkraft durchströmt wird. Nach dem zum Stand der Technik gehörenden Prinzip der Festphasensynthese (**Abb. 2**) werden so die einzelnen Produkte je Reaktor kovalent fixiert auf der Oberfläche des Trägermaterials und parallel durch eine Abfolge von Pipettieroperationen schrittweise aufgebaut. Während aller Syntheseschritte (Aufbaureaktionen, repetitive Schutzgruppenabspaltungen und Waschoperationen) bleiben die Produkte am Trägermaterial kovalent verknüpft und werden erst in einem oder mehreren letzten Reaktionsschritten vom Trägermaterial abgespalten und in Lösung gebracht;
12. die chemischen Bausteine (Monomere), die für den Aufbau der Produkte eingesetzt werden, als ASCII-Lettern codiert sind und die Produkte so als Abfolge von Aufbaureaktionen (Monomereinbaureaktionen) durch ASCII-Worte beschrieben sind; die Gesamtheit aller Produkte für ein Syntheseprogramm ist somit eine Liste von ASCII-Worten, die von einer geeigneten Software auf einem Steuerungsrechner in Ventilschaltung-, Dosierspritzenbewegungs- und Roboterarmbewegungsoperationen umgesetzt wird, wobei jeder Monomereinbau aus einer Folge von mehreren Reaktionsschritten und Schaltungsoperationen bestehen kann;
13. für die kovalente Verknüpfung der Produkte mit dem Trägermaterial nach 11. geeignete Verknüpfungsbausteine (Linker) vorgesehen werden, die eine selektive und schonende finale Abspaltung der Produkte erlauben (**Abb. 1**). Vorzugsweise wird für die Synthese von oligomeren Verbindungen, wie Oligonucleotide, Peptide usw., ein "universeller" Linker vorgesehen, an den auch die Bausteine der ersten Aufbaureaktion mit dem gleichen chemischen Reaktionstyp verknüpft werden können, der auch für die weiteren Aufbaureaktionen eingesetzt wird, wodurch für die ganze Synthese einer Verbindungsklasse nur ein Typ von Bausteinen gebraucht wird (z.B. nur Nucleosid-3'-phosphoramidite für die Synthese von 3'-OH-Oligonucleotiden);
14. vorzugsweise der Linker nach 13. bei den finalen Abspaltungsreaktionen erst in eine labile aber noch intakte Form überführt wird (safety-catch Linker), die dann durch eine milde chemische Behandlung, vorzugsweise eine pH-Veränderung, gespalten wird. Mit einem solchen Linkertyp lassen sich die kovalent fixierten Produkte einfach durch automatische Waschoperationen am Trägermaterial von chemischen Reagenzien reinigen und erst ganz zum Schluß aus den Reaktoren in eine der Reaktoranordnung komplementären Anordnung von Auffanggefäßen eluieren (**Abb. 9**);
15. die Zielprodukte der Synthesen mit einer als Affinitätslabel nutzbaren Gruppe versehen sind, über die die Zielprodukte an eine entsprechende Affinitätsphase gebunden werden können. Damit werden die aus den Reaktoren eluierten Produkte in eine der Reaktoranordnung komplementären Anordnung von Affinitätssäulen (**Abb. 9**) gereinigt. Nachfolgend werden die Zielprodukte durch einfache automatisierte Wasch- oder Abspaltungsoperationen aus den Affinitätssäulen in eine der Reaktoranordnung komplementären Anordnung von Auffanggefäßen eluiert;
16. die Bindungskapazitäten der Affinitätssäulen nach 15. so limitiert sind, daß auch bei unterschiedlichen Syntheseausbeuten je Reaktor eine gleiche Mindestmenge an Zielprodukt gebunden und eluiert wird, damit alle Produkte einer multiplen Synthese in äquimolaren Mengen erhalten werden.

Auf der Arbeitsfläche eines beispielhaften Pipettierroboters sind angeordnet:
- Ständer für Derivatelösungen, evtl. unter Schutzgas mit Septum
- Entnahmeports für Reagenzien, hier durch Ventile schaltbar
- Reagenzienflaschen unter Schutzgas
- Halterung für Reaktionssäulen

Die Reaktionssäulen bestehen aus Kunststoffröhrchen mit eingesetzten Fritten, die den eigentlichen Syntheseträger einschließen, den Träger in einer definierten Position fixieren oder selbst als Träger derivatisiert wurden. Als Röhrchen wurden hier handelsübliche Pipettenspitzen verwendet. Alternativ ist die Verwendung eines zusammenhängenden Spritzgußteils möglich, das einzelne Kavitäten mit Filterfritten enthält (z.B. von PolyFiltronics, Rockland, MA, USA, erhältlich).

Das Reaktionssystem kann im oberen Teil mit Schutzgas gespült werden. Eine Lochblende als Abdeckung kann auch bei kleinem Volumenstrom das Eindringen von Luft verhindern.

Der untere Teil des Reaktionssystems kann zur Absaugung der Reagenzien mit Vakuum beaufschlagt werden. In einer alternativen Anordnung kommt eine zweite, verschiebbare Lochblende zur Anwendung. Damit lassen sich die Löcher bei Bedarf verschließen und die Reaktionssäulen durch einen Überdruck von Schutzgas ausblasen.

Zur Dosierung von Reagenzien ist der Roboter mit einer Kanüle versehen, die mindestens zwei unabhängige Kanäle aufweist. Diese Kanäle treffen sich erst sehr kurz vor dem Auslaß. Über angeschlossene motorbetriebene Dosierspritzen lassen sich Reagenzien separat aufnehmen und simultan abgeben. Im einfachsten Fall besteht die Kanüle aus zwei konzentrisch angeordneten Rohren (vgl. Abbildung). Die Kanüle ist in der Längsachse federnd gelagert, um auch kleinste Volumina bis herunter zu 1 µl ohne Beschädigung der Trägerfritten auf diesen absetzen zu können.

Um die Synthesegeschwindigkeit zu erhöhen, kann eines oder mehrere der Reagenzien und Lösungsmittel auch über einen Verteilerkamm dosiert werden. Dieser Verteilerkamm wird entweder auch über eine Dosierspritze bedient, oder er ist über Ventile mit einem oder mehreren Vorratsbehältern unter Überdruck anschließbar. Der Verteilerkamm dosiert Reagenzien immer simultan auf eine Reihe von Reaktionssäulen. Die Synthese besteht nun aus einer Reihe von programmgesteuerten Übertragungen von Reagenzien und Lösungsmitteln aus den Vorratsbehältern in die Reaktionssäulen. Syntheseablauf und Art der Reagenzien sind im Prinzip bekannt und Stand der Technik.

Die Synthese beginnt mit der Festlegung der Sequenzen als Liste von ASCII-Zeichenketten im steuernden Computerprogramm. Der Syntheseablauf wird als Abfolge von Arbeitsschritten mit Angabe der Reagenzien, der jeweils zu übertragenden Volumina und der einzuhaltenden Reaktionszeiten definiert. Ein Beispiel für ein Ablaufprogramm ist im Anhang angegeben.

Das Gerät wird dann mit den notwendigen Reagenzien und Syntheseträgern bestückt. Konventionelle Träger nach dem Stand der Technik erfordern die Zuordnung zu den einzelnen Sequenzen, da der erste Baustein außerhalb des Geräts separat aufgebracht werden muß. Eine Vereinfachung erreicht man durch den Einsatz des erfindungsgemäßen universellen Trägers, bei dem der erste Baustein im Syntheseautomaten gekuppelt wird. Die Chemie dazu ist in der Anlage beschrieben.

Die Synthese beginnt typischerweise mit einem Waschschritt, der vorzugsweise über den Verteilerkamm ausgeführt wird. Zum Entfernen der Lösungsmittel wird das Absaugventil für eine bestimmte Zeit geschaltet und der untere Teil des Reaktionssystems mit Vakuum beaufschlagt. In dieser Zeit wird der Schutzgasstrom zum oberen Teil deutlich erhöht, um das Eindringen von Fremdluft weitgehend zu verhindern. Anschließend wird typischerweise eine Lösung zur Abspaltung der temporären Schutzgruppen über die Kanüle verteilt, z.B. Trichloressigsäure in Acetonitril. Die Kanüle wird zunächst in den geschlossener: Transferport gefahren, der gegen die Außenseite der Kanüle dicht abschließt. Dann wird das Ventil geöffnet und das Reagenz in einen der Kanäle der Kanüle aufgezogen. In der Regel wird mehr als die benötigte Menge aufgezogen, da es im Grenzbereich im Schlauch zwischen Kanüle und Dilutor zu Vermischungen kommen kann. Der Grenzbereich zwischen den Flüssigkeiten wird in der Regel durch eine zusätzlich aufgezogene Luftblase definiert. Das Ventil wird wieder geschlossen, und die Kanüle fährt die erste Reaktionssäule an. Die Kanüle wird gegebenenfalls auf der oberen Fritte oder dem Träger selbst aufgesetzt und das erste Aliquot des Reagenzes abgegeben. Die weiteren Positionen werden analog angefahren.

Anschließend wird der Überschuß im Schlauch verworfen und die Kanüle gespült.

Bei Reagenzien, die gemischt werden müssen, z.B. zur Aktivierung der Monomerbausteine, wird zunächst wie beschrieben eines der Reagenzien aufgenommen. Anschließend wird das zweite (und gegebenenfalls weitere) Reagenz in den zweiten Kanal der Kanüle aufgesaugt. Um schon für die erste Dosierung eine korrekte Mischung zu liefern, wird ein Teil des Überschusses zu Beginn durch gleichzeitiges Betätigen beider Dilutorspritzen verworfen. Die Kanüle fährt dann auf die vom Programm vorgegebenen Positionen, setzt auf der Fritte auf und dosiert das Reaktionsgemisch durch gleichzeitiges Betätigen der Dilutorspritzen. Dieses Verfahren ist der entscheidende Schritt der Synthese: Durch die Trennung der Reagenzien bis zur Dosierung auf den Träger wird die Konkurrenzreaktion zur Kupplung, die Hydrolyse durch Spuren von Wasser, verzögert. Damit ist die Synthese in einem eigentlich zur Atmosphäre offenen Reaktionssystem entgegen der vorherrschenden Meinung von Fachleuten doch möglich.

Der weitere Syntheseablauf besteht aus einer Abfolge ähnlicher Schritte mit wechselnden, vom Programmablauf festgelegten Reagenzien.

Im Anschluß an die Synthese erfolgt die bekannte Entschützung und Abspaltung der Produkte vom Träger, z.B. durch Inkubation mit konzentrierter Ammoniaklösung.

Alternativ läßt sich ein erfindungsgemäßer "Safety-Catch-Linker" verwenden, der bei Abspaltung der Schutzgruppen erhalten bleibt und lediglich in einen labileren Zustand gebracht wird. So können alle Reagenzien und Nebenprodukte ausgewaschen werden, bevor in einem separaten Schritt die Syntheseprodukte vom Träger abgespalten werden. Der Einsatz des Safety-Catch-Linkers erleichtert die Syntheseaufarbeitung erheblich, da Ammoniak und Nebenprodukte leichter abgetrennt werden können als es bisher möglich war.

Die Aufarbeitung wird ebenfalls erleichtert durch die Anorndung der Reaktionsgefäße in einem Standardformat, z.B. dem von Mikrotiterplatten.

Da die Reaktionen während der Synthese niemals vollständig ablaufen, ist es durchaus üblich, wenn auch nicht immer notwendig, die Produkte zu reinigen. Hier bietet sich das von Blöcker und Frank beschriebene Verfahren (9A) an. Alternativ lassen sich auch andere bekannte Affinitätsreinigungen anwenden, z.B. über die Interaktion von Biotin und Avidin (**17A, 17B**).

Die meisten Anwendungen erfordern eine relativ genau bestimmte Menge an Syntheseprodukt. Im Fall der Oligonucleotide für Sequenzierreaktionen ist das nur ein Bruchteil der Syntheseausbeute. Hier läßt sich die Ausbeute über alle Syntheseansätze erfindungsgemäß durch Verwendung einer limitierenden Menge an Affinitätsmatrix nivellieren. Die Menge an Affinitätsmatrix wird gut definiert und in jedem Fall deutlich mehr markiertes Syntheseprodukt aufgegeben als der Bindungskapazität entspricht.

Die anschließend eluierten Mengen an Syntheseprodukt sind dann in etwa gleich (Anlage 9B). Die Kombination von Affinitätsreinigung und Aliquotierung ist neu und erspart aufwendige Konzentrationsmessungen.

Durch den modularen Aufbau des Syntheseautomaten wird es möglich, Synthese, Abspaltung, Reinigung und Aliquotierung in einem Gerät zu vereinen. Bei Anwendung des Verfahrens nach EP 0 174 525 kann ein Affinitätsträger aus Polymermaterial, z.B. Polystyrol, Polyethylen oder Modifikationen davon, benetzt werden. Das Material kann so gewählt werden, daß es gegenüber der Synthese von Schutzgruppenabspaltung inert ist. Es läßt sich dann sogar als Fritte unter dem Syntheseträger anordnen, was die Aufarbeitung gegenüber dem Stand der Technik erheblich vereinfachen würde.

### A) Chemische Voraussetzungen zur hochparallelen DNA-Synthese

Das Arbeitsprinzip des konzipierten multiplen Syntheseautomaten sieht ein Raster von einfachen kleinen Reaktoren vor, in denen kleine poröse Membranfritten das Trägermaterial für die Synthese darstellen. Der Synthesemaßstab je Reaktor soll an die Anwendung als Sequenzier-Primer optimal angepaßt sein und im Bereich 1 bis 10 nanomol liegen. Das Konzept zur chemischen Derivatisierung des Trägermaterials für den Einsatz zum schrittweisen Aufbau von Oligonucleotiden ist in **Abb. 1A** dargestellt.

Es wurden zunächst ausreichend empfindliche Detektionsreaktionen etabliert, um die einzelnen Derivatisierungsschritte quantitativ im nanomol-Maßstab verfolgen zu können:
- für Hydroxyfunktionen: Tritylierung mit Dimethoxytritylchlorid in Pyridin gefolgt von der sauren Detritylierung mit Dichloressigsäure in Dichlorethan und photometrische Bestimmung des Dimethoxytritylkations;
- für Aminofunktionen: Anfärbung mit dem anionischen Farbstoff Bromphenolblau in Dimethylformamid, gefolgt von der basischen Dissoziation und photometrischen Bestimmung des Bromphenolblau-Anions.

Verschiedenste, kommerziell erhältliche Membranmaterialien auf Polyolefinbasis wurden dann auf Eignung untersucht. Je nach Herstellung weisen diese Materialen schon eine unspezifische oxidative Alterung der Oberfläche mit Hydroxyl-, Keton-, Aldehyd- und Carboxylgruppen auf (**Abb. 2**). Daher wurde eine Prozedur entwickelt, die diese Polymeroberfläche zunächst reduktiv "säubert" und anschließend durch selektive und leicht steuerbare Oxidation ausschließlich die hier gewünschten Hydroxylgruppen generiert (**Abb. 3**). Damit kann jetzt Frittenmaterial mit vorbestimmter und für den Einsatz zur Oligonucleotidsynthese geeigneter Funktionalität hergestellt werden. Eine Vielzahl von chemischen Umsetzungen dieser sowie auch anderer hydroxylierter Materialien (z.B. Cellulosepapiere) wurde durchgeführt, um geeignete "Spacer" und "Linker" aufzubringen (**Abb. 4**). Die so erhaltenen Fritten wurden in einem kommerziellen Syntheseautomaten für Oligonecleotidsynthesen erfolgreich eingesetzt (**Abb. 5**) und stehen jetzt für Tests im Reaktormodul des neuen Automaten zur Verfügung.

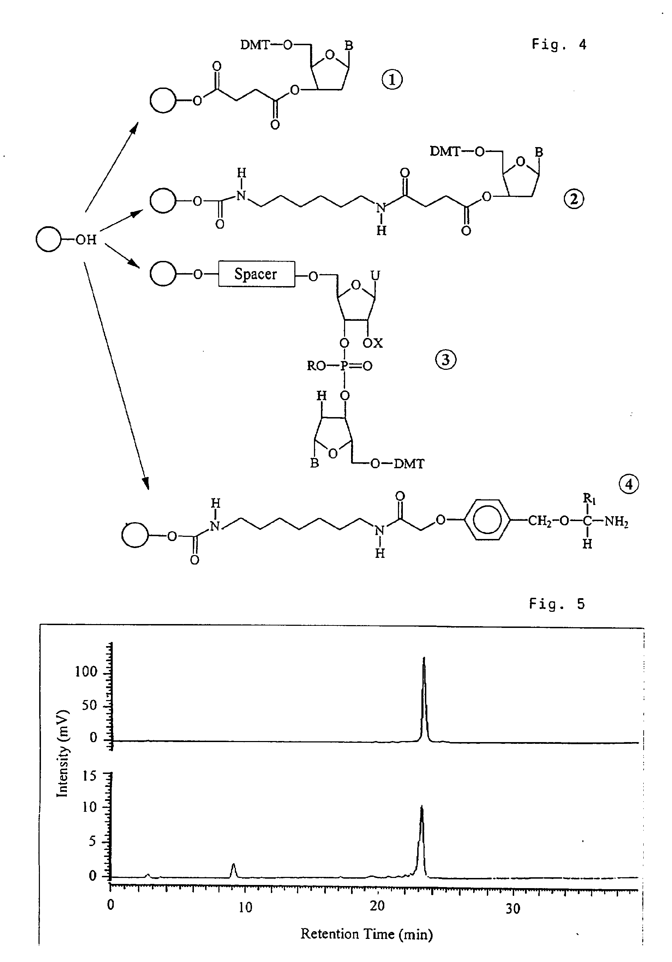

Idealerweise sollte das nach Abbildung 1A vorbereitete Trägermaterial (Träger-Spacer-Linker) universell für jede Oligonucleotidsequenz einsetzbar sein, so daß keine individuelle Konfigurierung des Syntheserasters notwendig ist. Dies ist durch die konventionelle Beladung in einer separaten Reaktion mit 3'-Nucleosidsuccinaten nicht gegeben (jede Sequenz erfordert einen der vier verschiedenen beladenen Träger). Deshalb soll ein universeller "Linker" implementiert werden, auf dem die Oligonucleotidsynthese direkt mit einem ersten Nucleotidbaustein beginnen kann. Das Konzept der intramolekular spaltbaren Phosphodiester nach Köster und Heyns (Tetrahedron Letters 1972, 1531) bzw. Gough et al. (Tetrahedron Letters 1983, 5321) wurde hierfür herangezogen und geeignete Linkerbausteine hergestellt und am Trägermaterial verankert (**Abb.4**, Verbindungstyp 3). In Modellsynthesen konnte erfolgreich gezeigt werden, daß das Konzept funktioniert.

Ein weiterer Aspekt der konzipierten Synthesetechnologie ist die integrierte parallele Reinigung und Lagerung der Oligonucleotide. Durch Verwendung einer eigens dafür zu entwickelnden "safety-catch"-Verankerung auf der Trägeroberfläche soll eine trägerfixierte Entschützung und Reinigung realisiert werden. Hierfür wurde ein chemisches Konzept entwickelt, das eine Modifikation des universellen Linkers darstellt (**Abb. 4**, Verbindungstyp 3). Entsprechende Modellverbindungen wurden hergestellt, mit denen das Konzept erfolgreich auf Funktionsfähigkeit überprüft wurde.

### B) Experimentalversion einer DNA-Synthesemaschine

Eine erste Version des Gerätes wurde anhand des ursprünglichen Konzeptes aufgebaut und die notwendige Steuerungssoftware extern erstellt (**Abb. 6 bis 9**).

### C) Beispiele für Quantifizierung (und Reinigung) der produzierten Oligonucleotide

Je nach Basensequenz der synthetisierten Oligonucleotide erhält man unterschiedliche Gesamtausbeuten. Das folgende Verfahren ermöglicht es, eine einheitliche definierte Menge der verschiedenen Oligonucleotide zu erhalten. Gleichzeitig werden die Oligonucleotide dabei gereinigt.
1. Als Material für die Quantifizierung dient ein hydrophobes Polyolefin-Pulver (PE, PP oder PTFE). Definierte Mengen dieses Pulvers werden in Sterilfilter-Spitzen (Fa. Eppendorf) oder in entsprechenden Spitzen mit Fritten gegeben (z.B. 20 mg, 10 mg, 5 mg oder 2,5 mg). Vorbereitet wird das Pulver durch Waschen mit Acetonitril (je 3 x 250 µl) und anschließend mit 1 M Triethylaminacetat-Puffer (TEAA) (je 3 x 250 µl).
2. Die mit dem PRIME96-Syntheseroboter dargestellten Oligonucleotide, die noch die hydrophobe Trityl-Schutzgruppe besitzen, werden vom Träger abgespalten. Man erhält so die Lösung A (in diesem Beispiel: 200 µl 33% NH₃-Lösung). Diese Lösung A wird 1:1 mit Wasser verdünnt und auf die vorbereiteten Säulen gegeben und langsam mit Hilfe einer Spritze durchgedrückt.
3. Es wird mit 2,5% NH₃ (aq) (je 3 x 250 µl) und Wasser (je 3 x 250 µl) gespült.
4. Die Trityl-Schutzgruppe wird nun mit 2% Trifluoressigsäure (aq) (je 3 x 250 µl) abgespalten. Nach 5 min wird mit Wasser (je 3 x 250 µl) gespült.
5. Eluiert wird da quantifizierte und gereinigte Oligonucleotid mit 20% Acetonitril (aq) (je 3 x 250 µl).

### Beispiel:

Aufgetragen wurden 7,2 OD (ca. 40 nMol) eines gemischten 18'mers

| mg Quantifizierungs Harz* | Auftragseluat (OD₂₆₀) | TFA-Eluat (OD₂₆₀) | End-produkt (OD₂₆₀) | Wiederfindungsrate (%) |
|---|---|---|---|---|
| 2,5 | 5,5 | 0,9 | 0, 427 | 94 |
| 5 | 5,22 | 0,9 | 0,968 | 98 |
| 10 | 4,3 | 0,7 | 1,93 | 95 |
| 20 | 3,32 | 0,4 | 2,68 | 88 |
| • Micropure, BTI Technologies, San Raffael, CA, USA | | | | |

Als "Faustformel" ergibt sich aus dieser Testreihe, daß ca. 1 nMol Oligonucleotid pro mg Quantifizierungsharz gebunden wird. Die Quantifizierung beruht darauf, daß eine definierte Menge des zunächst noch tritylierten Oligonucleotids pro mg des eingesetzten Reinigungsmaterials gebunden wird. Da die Menge des mit dem PRIME96 jeweils sythetisierten Oligonucleotids stets größer ist als die Menge, die vom Reinigungsmaterial gebunden werden kann, ist so auf einfache Weise eine Quantifizierung möglich.

### D) Beispiel für Trägermaterial für die Festphasensynthese mit einem Syntheseroboter

### Innovatives Konzept:

Unser innovatives Synthesekonzept basiert darauf, daß die verschiedenen Reaktionslösungen allein durch Kapillarkräfte an einem festen Träger haften und mit diesem reagieren können. Dabei kann also so auf anfällige und teure Ventilsysteme verzichtet werden.

Dieses kann zum einen dadurch realisiert werden, daß man zwischen zwei inerte Fritten ein konventionelles Trägermaterial einbringt. Noch besser ist es jedoch, einen Träger zu entwikkeln, der bereits eine definierte poröse Struktur besitzt, um diesen dann zu funktionalisieren. Das letztere Verfahren bietet dabei folgende Vorteile:
- kommerziell erhältliches Trägermaterial mit definiertem macroporösem Porendurchmesser
- reproduzierbare Herstellung
- große Oberfläche
- gute Durchströmungseigenschaften
- definierte Struktur
- mechanisch stabil
- chemisch weitgehend inertes Grundmaterial, nur die Oberfläche wird derivatisiert

Ein derartiges neues Trägersystem haben wir hier entwickelt, wobei besonders die poröse Struktur des Ausgangsmaterials eine Funktionalisierung mit bekannten Verfahren ausschloß.

### Funktionalisierung von Polyolefin-Fritten (am Beispiel von Polyethylen- und Polypropylen)

### 1) Reduktion

Bedingt durch den Herstellungsprozeß (Sinterung) sind die kommerziell erhältlichen Polyolefin-Fritten bereits oxidiert (Carboxy-, Carbonyl-, Hydroxylgruppen). Um hier ein einheitliches Startmaterial herzustellen, ist zunächst eine Reduktion nötig. Dazu werden in einem Dreihalskolben mit Rückflußkühler 50 mMol LiAlH₄ vorgelegt und 50 ml trockener Diethylether zugetropft. Danach werden 1 g PE-Fritten hinzugefügt und 6 h unter Rückfluß erhitzt. Hiernach wird zunächst mit feuchtem Ether der Überschuß von LiAlH₄ hydrolysiert und der Niederschlag mit 10% H₂SO₄ wieder aufgelöst. Es wird 3 x mit 100 ml Wasser, dann mit 2 x 100 ml 10% NaHCO₃, danach wieder mit 3 x 100 ml Wasser und 1 x 100 ml MeOH gewaschen und im HV getrocknet.

### 2) Direkte Hydroxylierung

1 g der reduzierten Polyolefin-Fritten werden zu einer Lösung von 10 ml H₂O₂ (30% aq) und 100 ml Trifluoressigsäure gegeben. Unter Rückfluß wird 15 bis 60 min erhitzt.
Je nach Reaktionszeit ergeben sich Beladungen von 250 nMol OH/g (15 min) bis 5 µMol OH/g (60 min). (Die Beladung mit OH-Gruppen wurde über DMT-Kopplung bestimmt.)
Die hier verwendete Methode zur Hydroxylierung ist in der Literatur bis jetzt nur für Alkane und Cycloalkane beschrieben.

Die so hergestellten porösen hydroxylierten Träger sind universell für alle Festphasensynthesen einsetzbar.

### 3) Kopplung des ersten Bausteins (Startnucleosids) an den Träger

### Beispiel 1 (s.a. Abb. 4, Anlage 1):

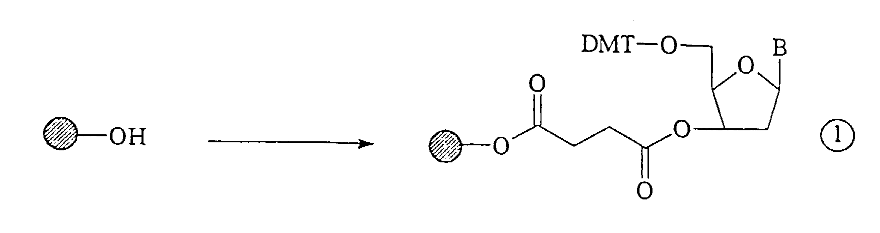

20 µMol DMT-dT^{an}-Succ werden in 100 µl DMF gelöst und 31 µMol N,N'-Diisopropylcarbodiimid (DICD) zugemischt. Nach 10 min werden 25 µMol Methylimidazol zugemischt und 60 mg der hydroxylierten Polyolefin-Fritten (Darstllung s.o.) unter Stickstoff zugegeben. Nach 24 Stunden bei 25°C werden die Fritten nacheinander mit DMF, Pyridin und Methylenchlorid gewaschen.

### Beispiel 2: Einführung eines Spacers (s.a. Abb. 4, Anlage 1):

Einführung des Hexamethylen-Spacers:

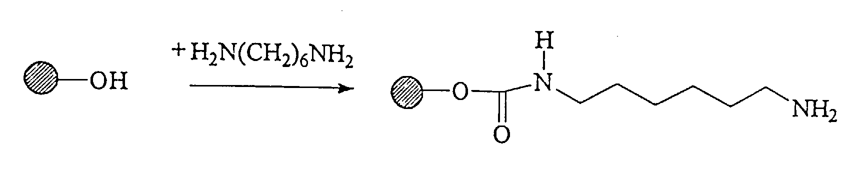

In 5 ml einer 0,3 M Lösung von 1,1'-Carbonyldiimidazol in DMF werden 180 mg im HV getrocknete hydroxylierte Polyolefin-Fritten (Darstellung s.o.) unter Stickstoff 6 Studnen bei RT geschüttelt. Danach werden die Fritten 3 x mit je 50 ml DMF gespült und danach in 5 ml einer 0,3 M Lösung von 1,6-Diaminohexan in DMF 18 Stunden geschüttelt. Anschließend werden die Fritten mit DMF, Methanol, Aceton und Ether gewaschen und im HV bei RT getrocknet.

Kopplung des Startnucleosids:

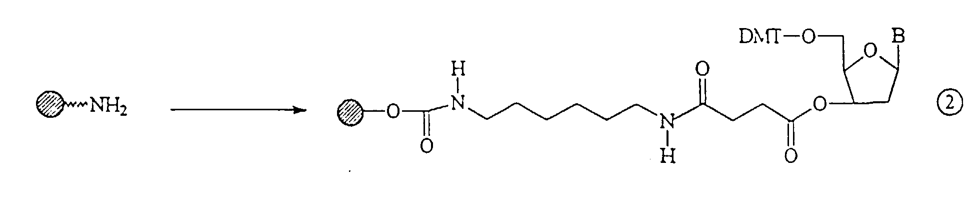

Durchführung analog Beispiel 1, jedoch ohne Methylimidazol.

### Funktionalisierung von PTFE

PTFE-Fritten werden mit Natriumnaphthalid behandelt und anschließend hydrolysiert. Bereits hier erhält man hydroxylierte PTFE-Fritten. Durch eine anschließende Hydroborierung kann man die Konzentration von Hydroxygruppen auf dem Träger noch erheblich steigern.

Weiteres Trägermaterial (im PRIME96 getestet)
- Cellulose (s. Anlage 12)
- kommerzielles Trägermaterial (Fa. Pharmacia) als Pulver zwischen zwei Fritten (s. Anlage 11)

### E) Beispiele für Linker

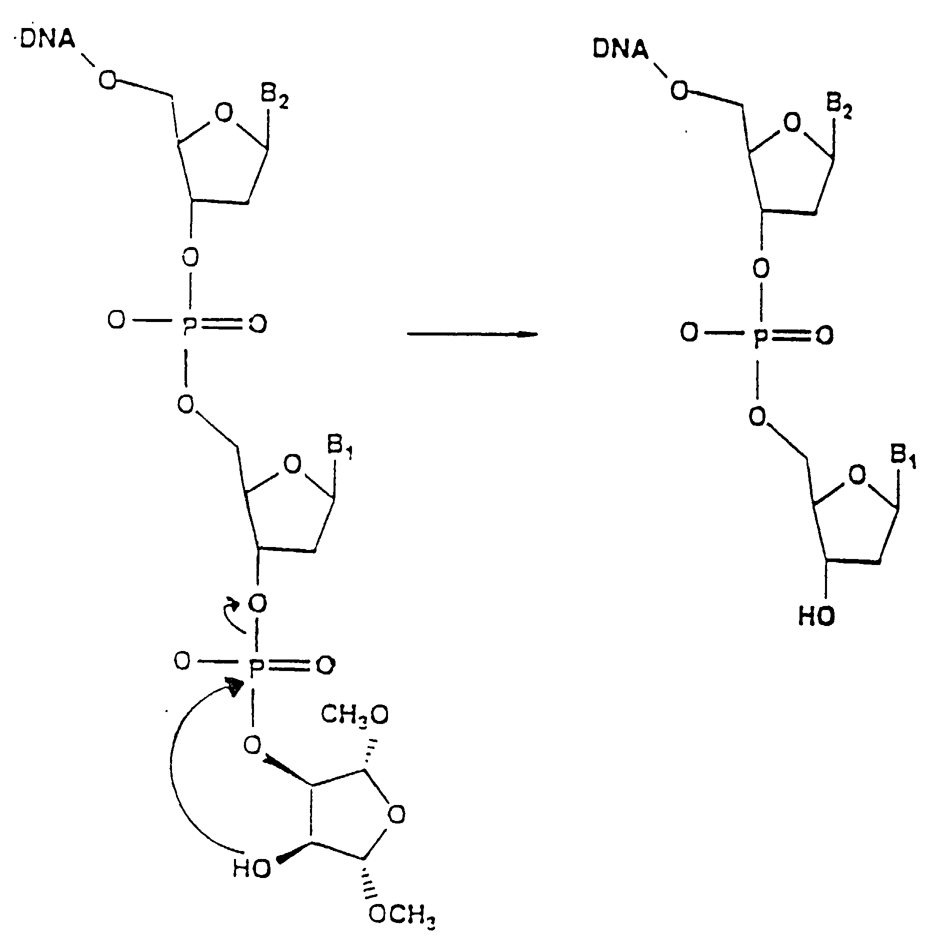

H. Köster und K. Heyns (1972)^, Tetrahedron Letters 1972, 1531
G.R. Gough, M.J. Brunden und P.T. Gilham (1983), Tetrahedron Letters 1983, 5321
Scott, P. Hardy, R.C. Sheppard und M.J. McLean (1994); in: Solid Phase Synthesis (R. Epton, Hg.), Mayflower Worldwide Ltd., Birmingham, UK, S. 115

Neue universelle Linker für die Synthese von Oligonucleotiden an fester Phase (Phosphoramiditchemie):

Folgende Linkersysteme wurden hergestellt und getestet:

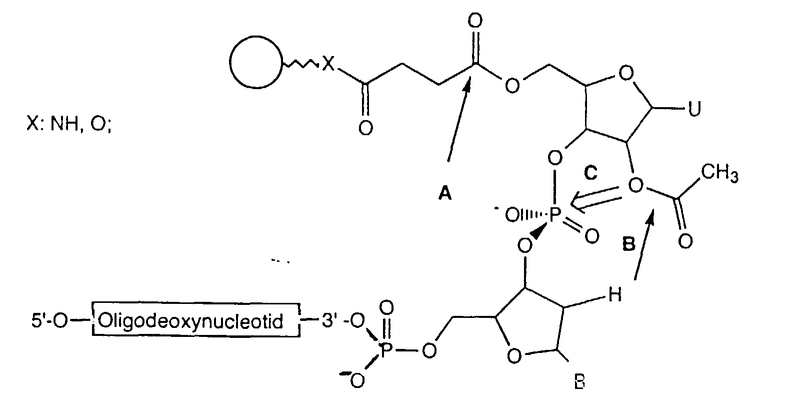

### Typ 1a : Abspaltung vom Träger und gleichzeitige Hydrolyse des Linkermoleküls:

A und B: schnelle Esterhydrolyse durch NH₃ oder LiOH; C: langsame Phosphodiestercyclisierung durch Säure oder Base;

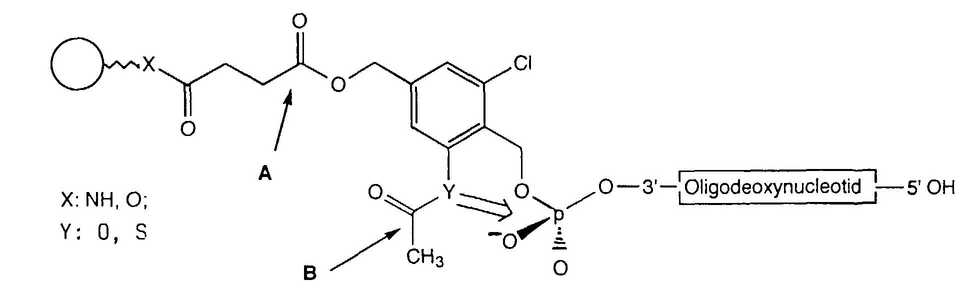

### Typ 1b: Abspaltung vom Träger und gleichzeitige Hydrolyse des Linkermoleküls:

A und B: schnelle Esterhydrolyse druch NH₃ oder LiOH; C: beschleunigte Phosphodiestercyclisierung durch Säure oder Base;

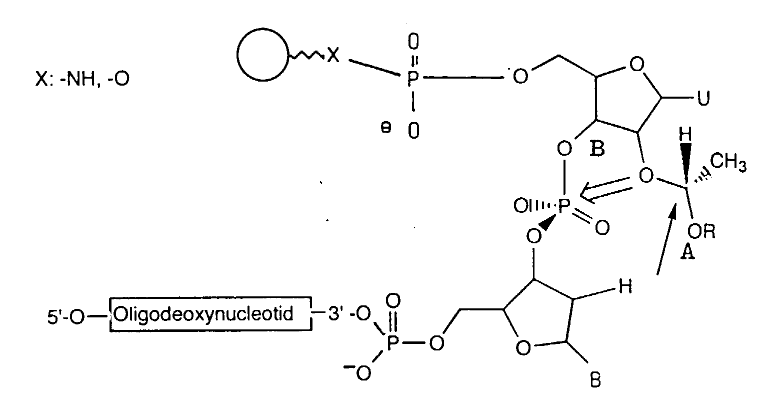

### Typ 2: Irreversible Verankerung des Linkermoleküls auf der Festphase;

Abspaltung des Linkers durch 2-Stufenmechanismus;
A: schnelle saure Acetalhydrolyse (verd. ACOH/H₂O);
B: langsame Phosphodiestercyclisierung (LiOH);

Neue universelle Linker für die Oligonucleotidsynthese (Phosphoramiditchemie):

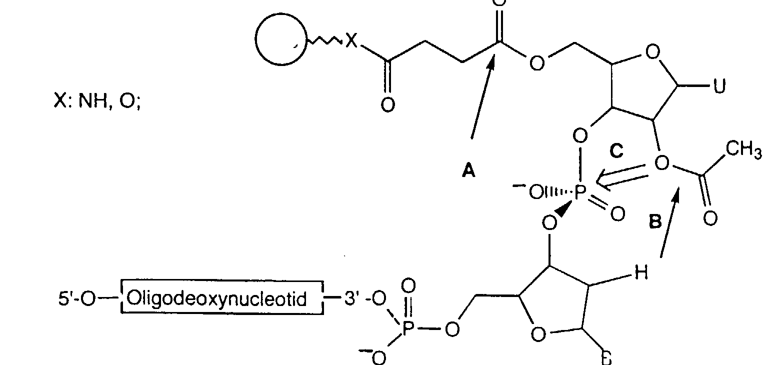

### Typ 1a: Abspaltung vom Träger und gleichzeitige Abspaltung des Linkerbausteins:

A und B (schnelle Esterhydrolyse, NH₃ oder LiOH);
C (Langsame Phosphodiestercyclisierung, NH₃ oder LiOH);

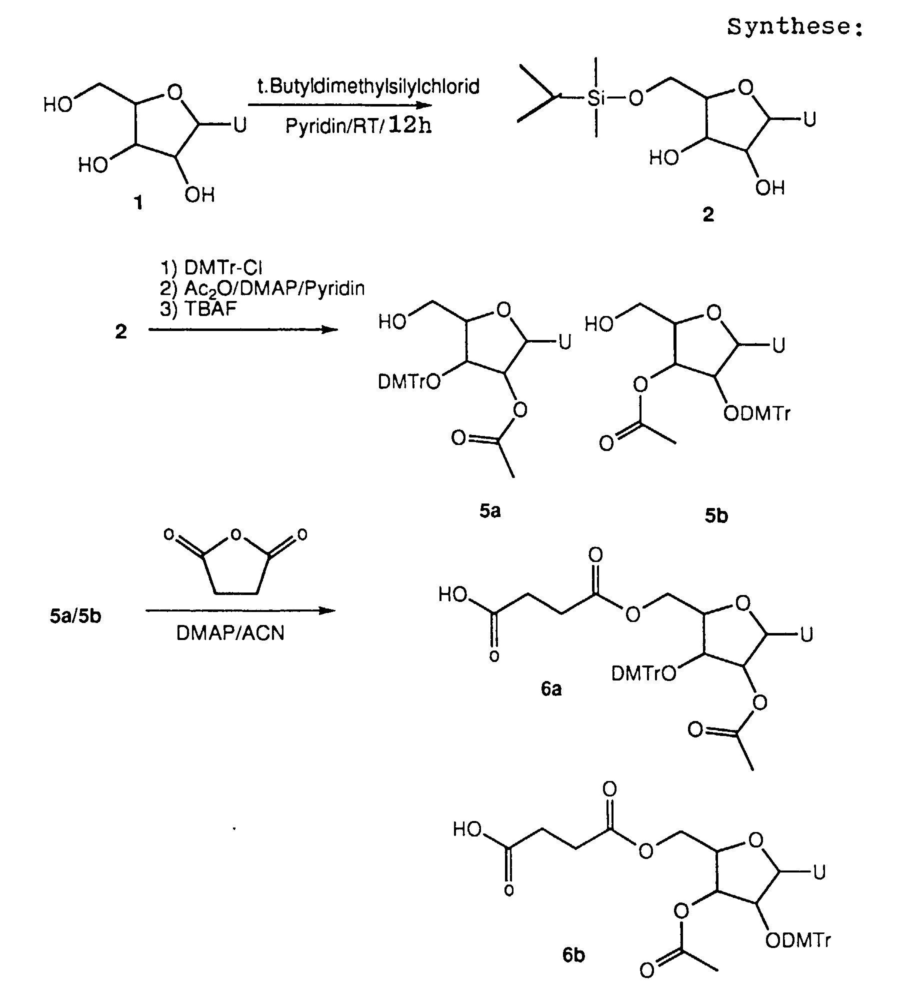

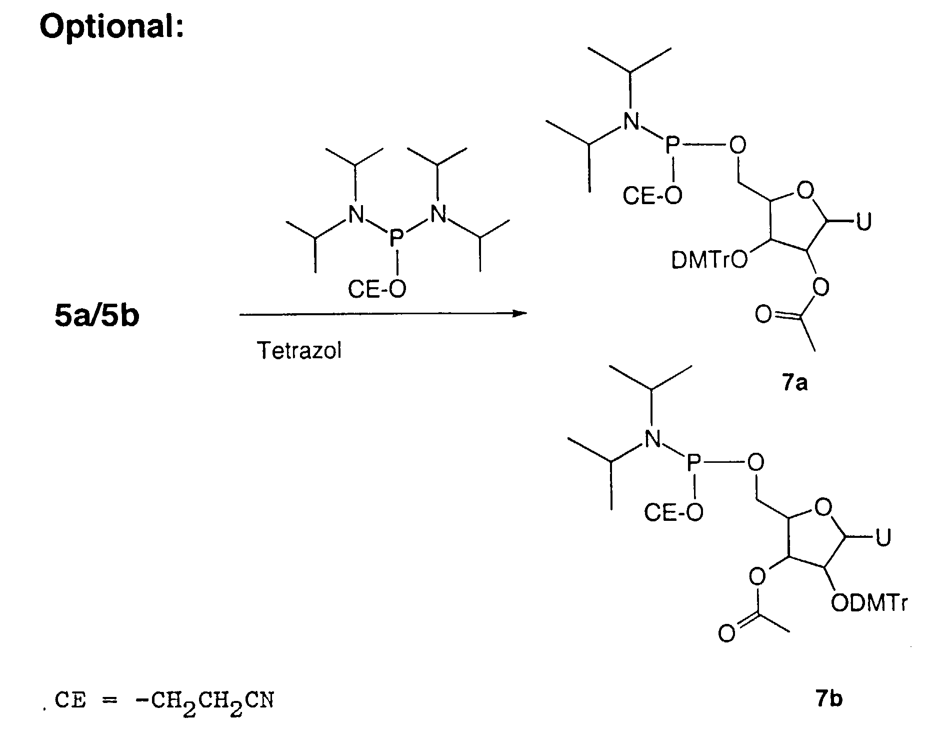

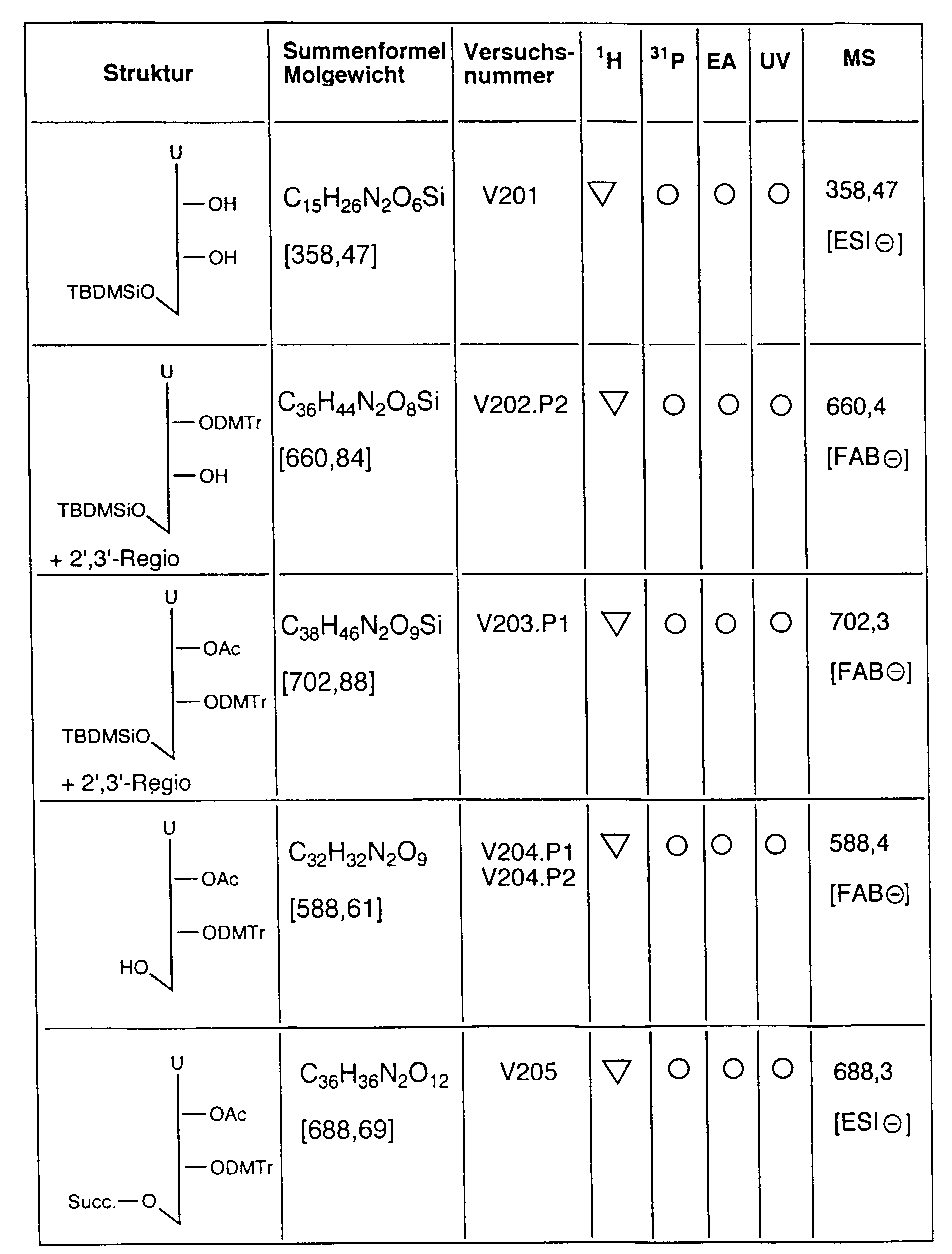

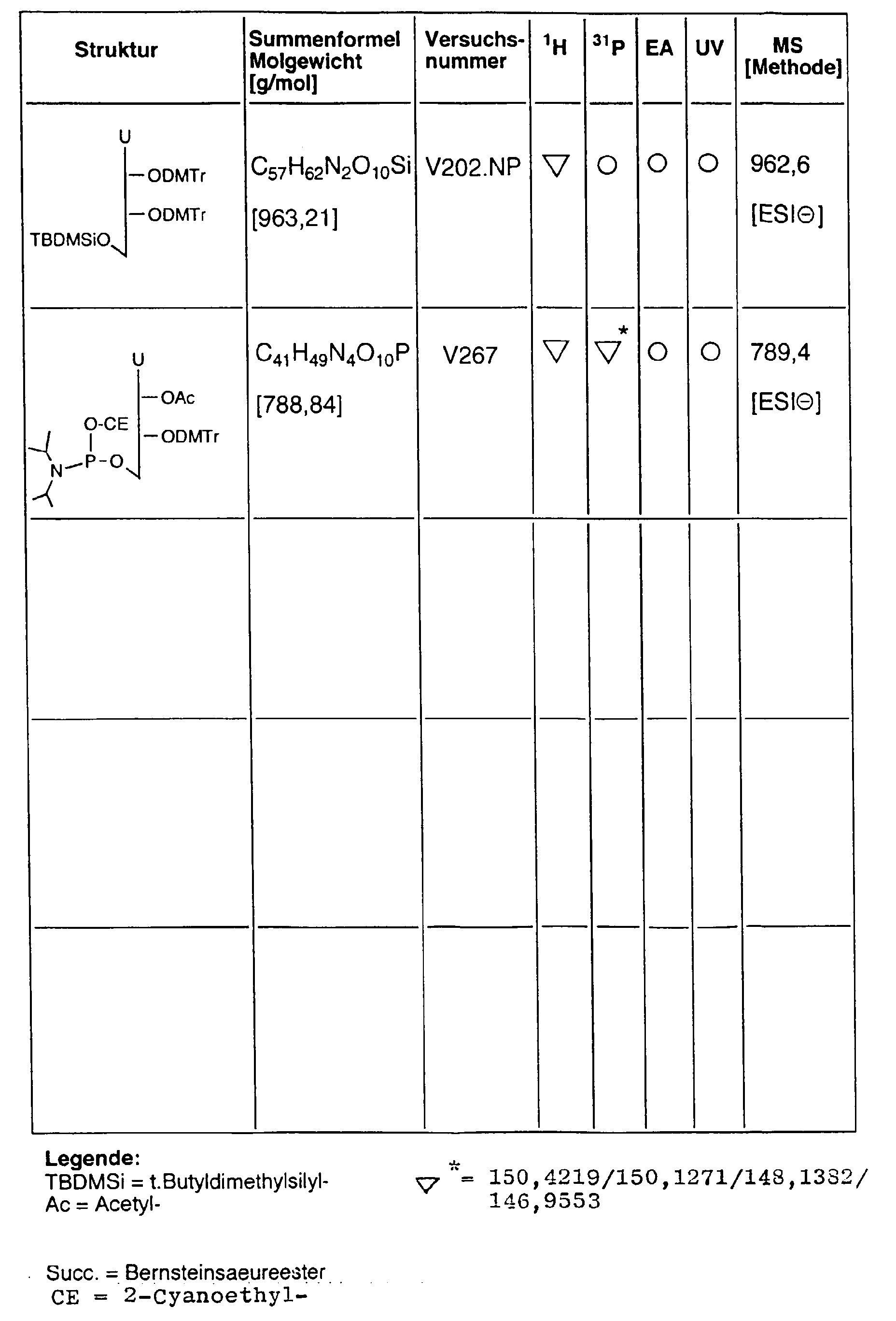

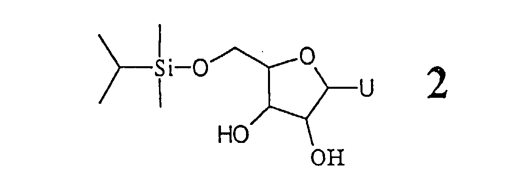

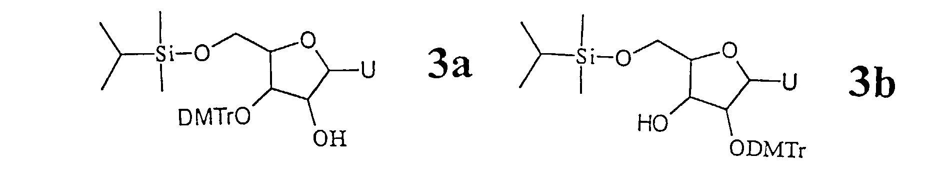

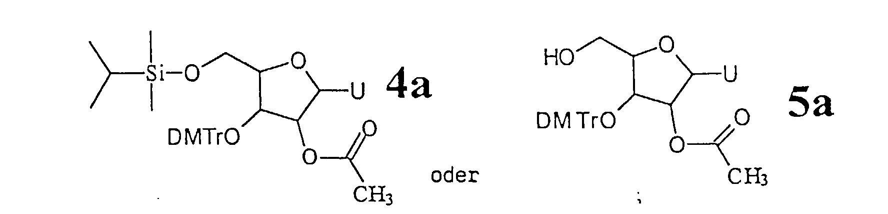

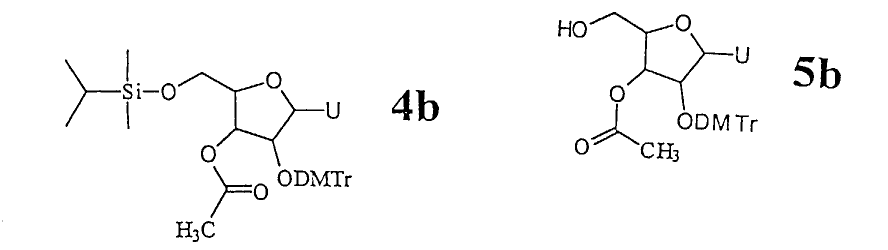

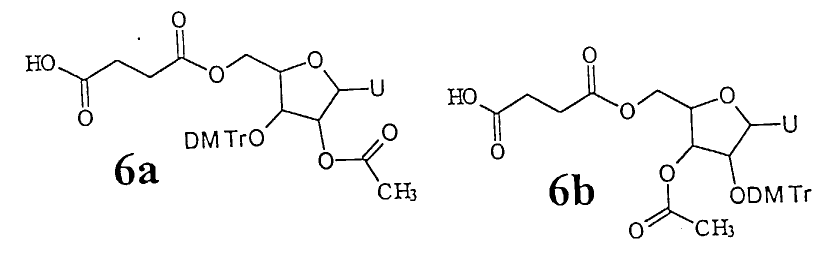

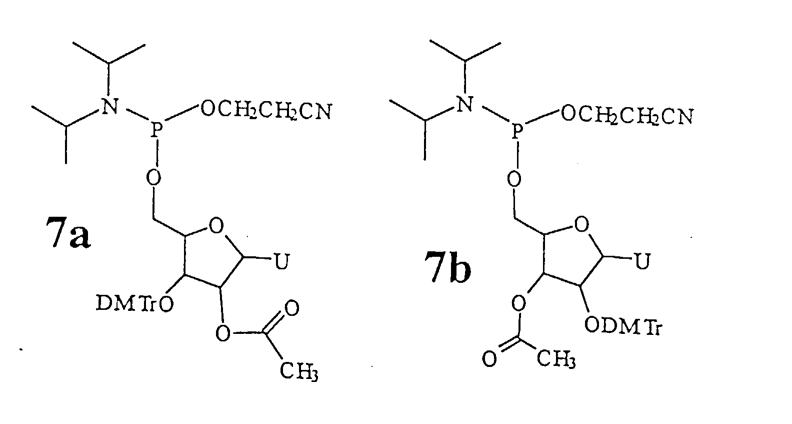

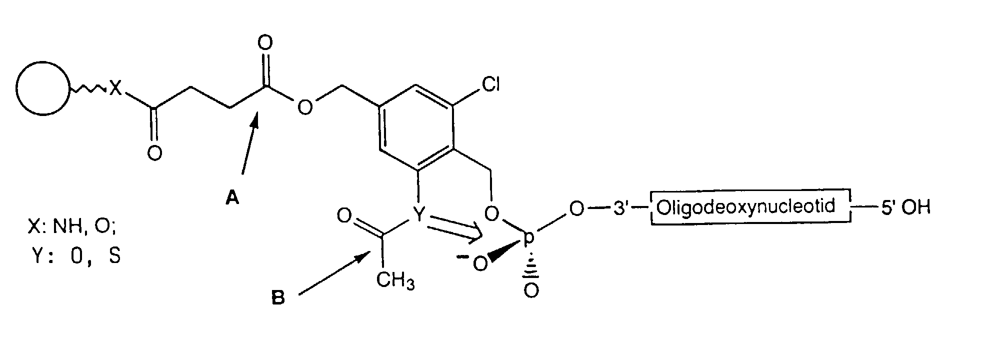

### Typ 1b: Abspaltung vom Träger und gleichzeitige Abspaltung des Linkermoleküls:

A und B (schnelle Esterhydrolyse, NH₃ oder LiOH);
C (beschleunigte Phosphodiestercyclisierung durch Phenolat- oder Thiophenolatbildung mit NH₃ oder LiOH))

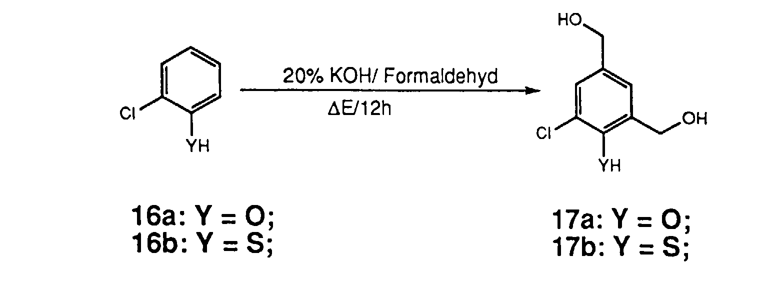

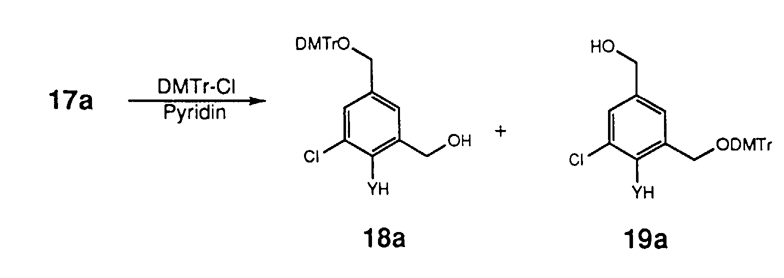

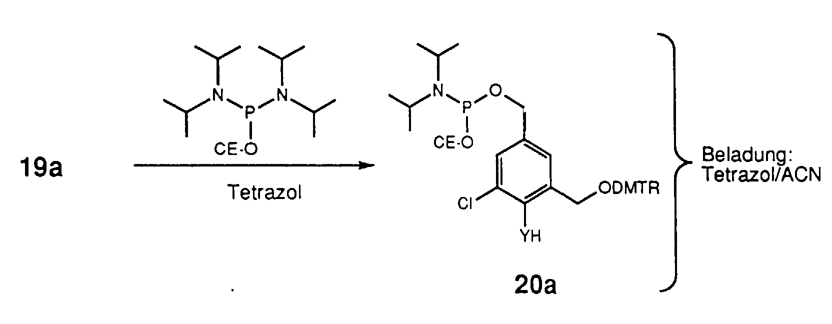

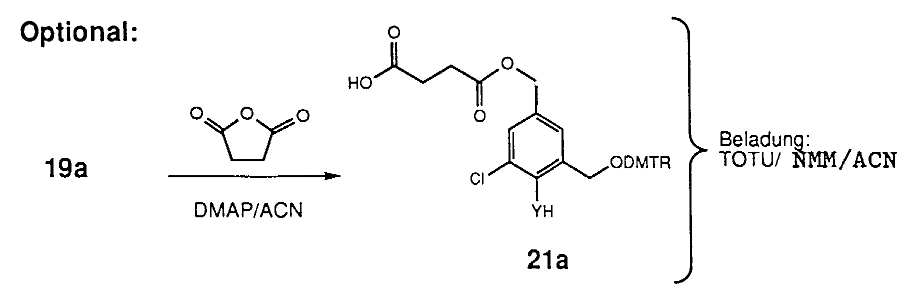

### Capping:

Nach Beladung des Trägermaterials erfolgt die Acylierung (Capping) der phenolischen HO- oder HS-Funktion durch Umsetzung mit Essigsäureanhydrid oder Pivaloylchlorid unter DMAP-Katalyse;

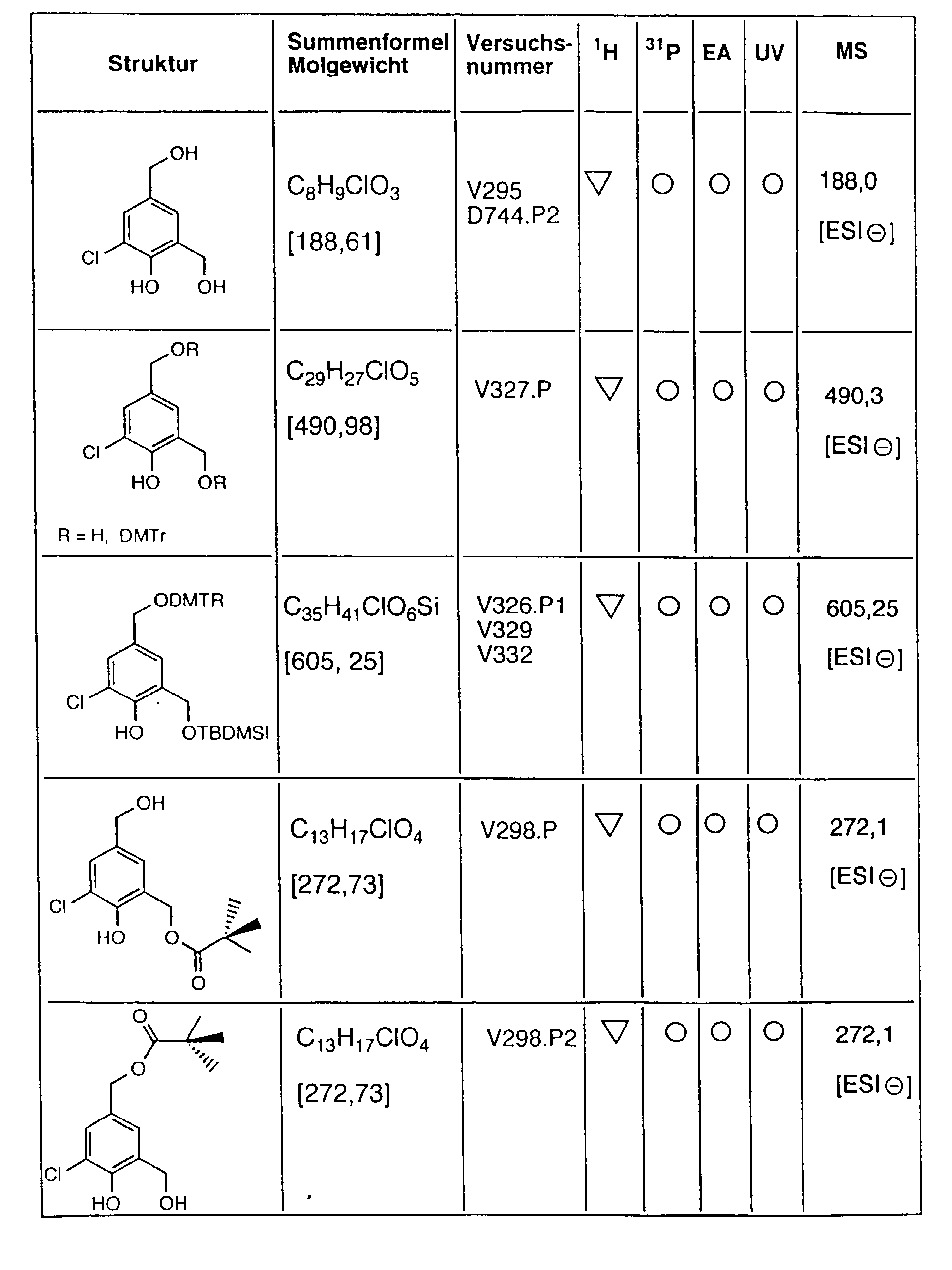

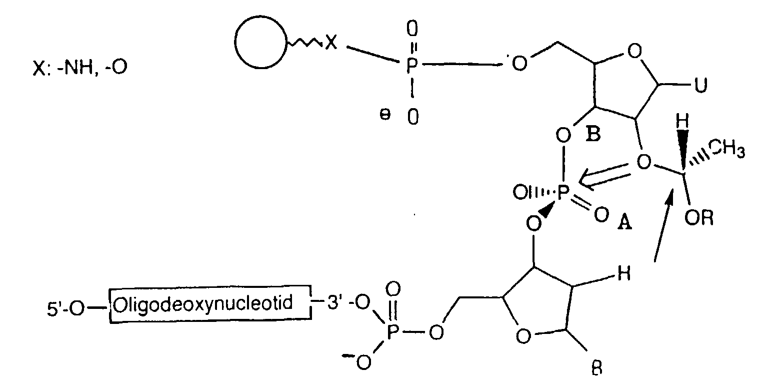

### Typ 2: Irreversible Verankerung des Linkermoleküls auf der Festphase;

Abspaltung des Linkers durch 2-Stufenmechanismus
A: schnelle saure Acetalhydrolyse (AcOH);
B: langsame Phosphodiestercyclisierung (LiOH);

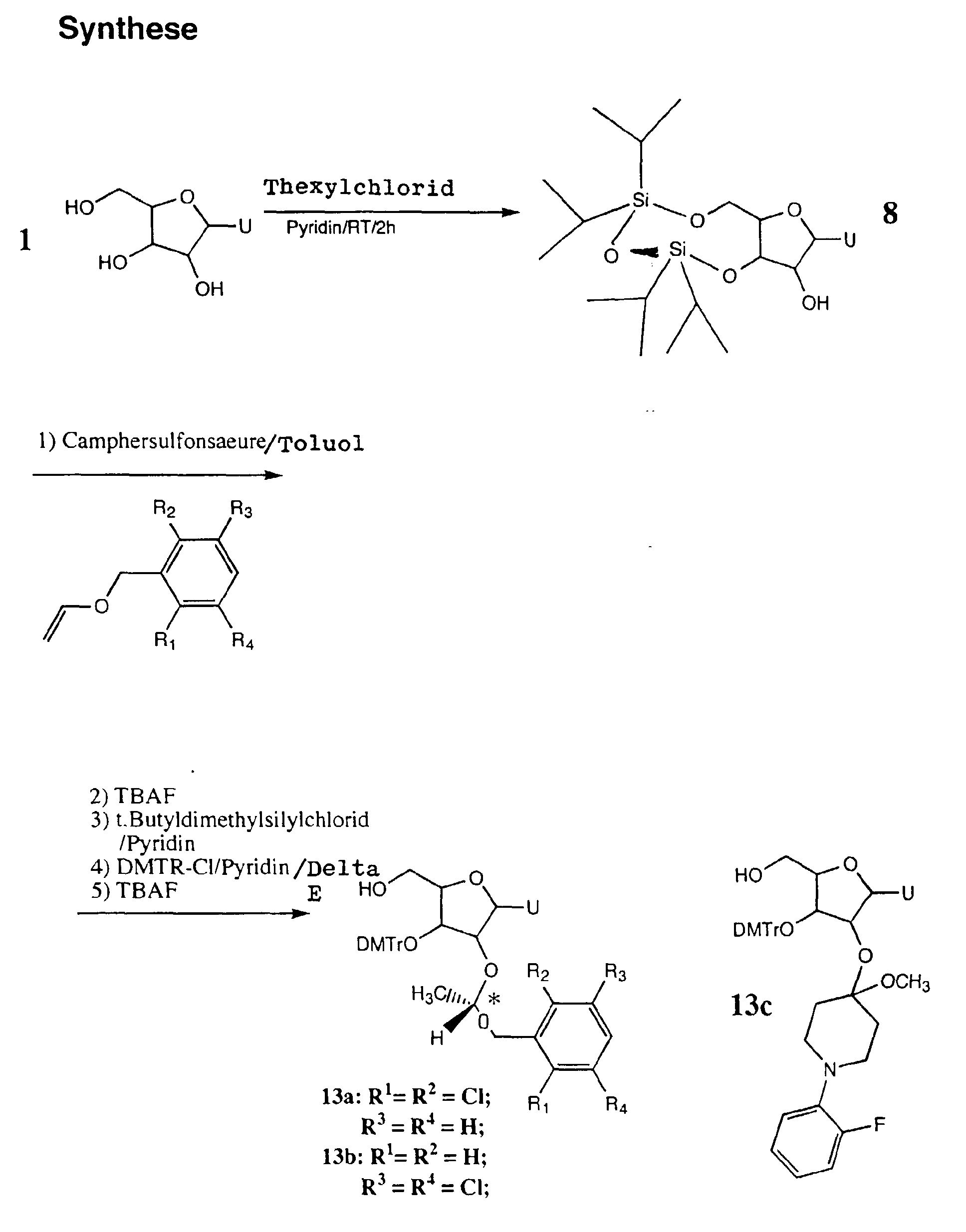

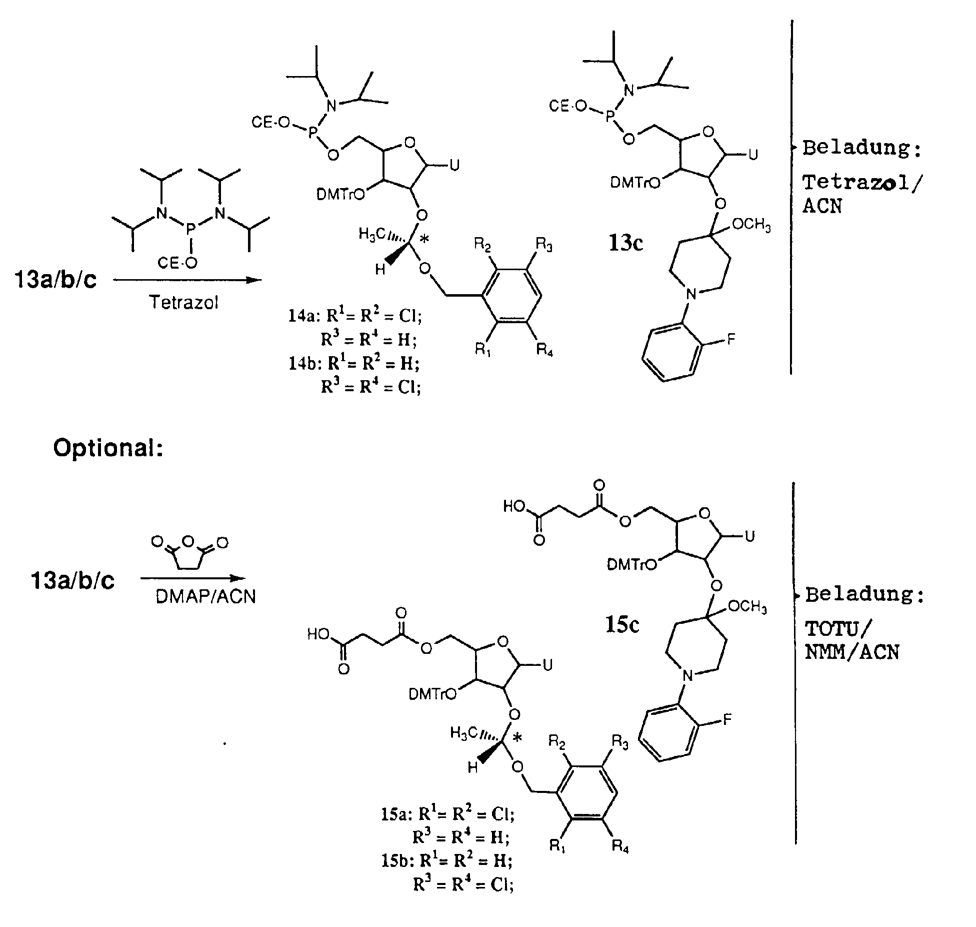

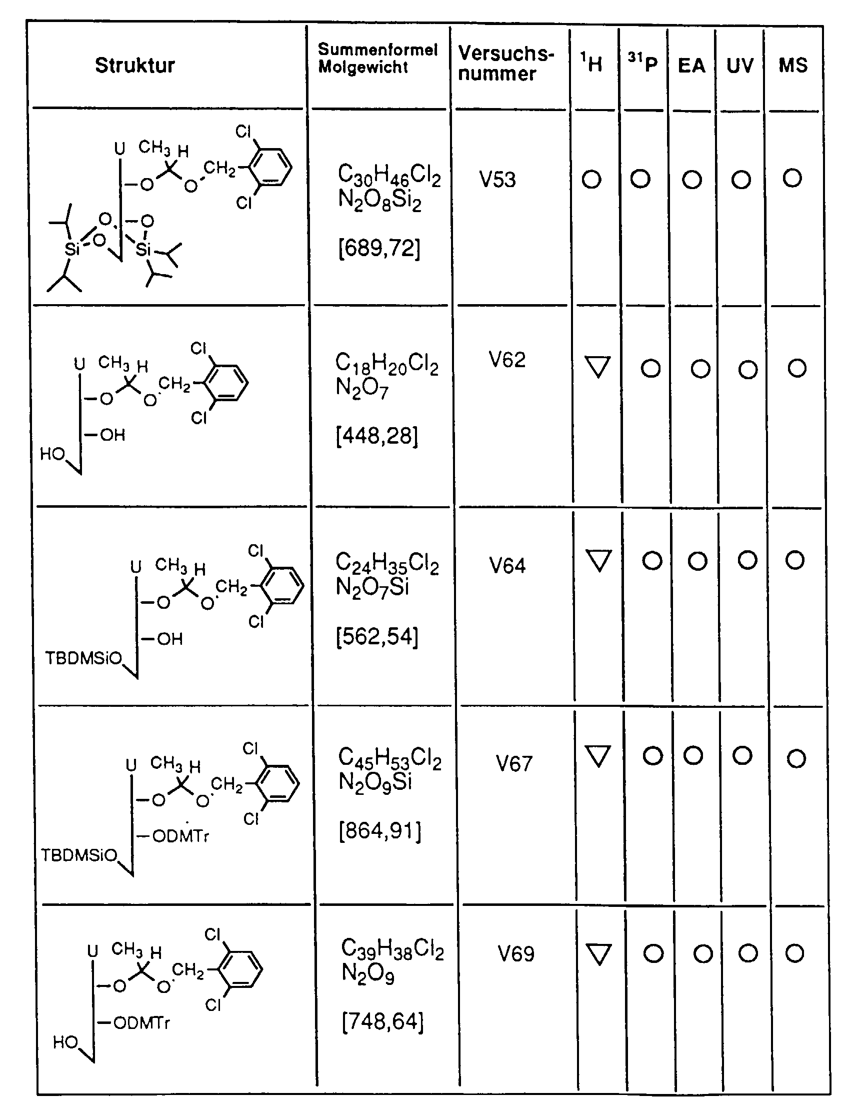

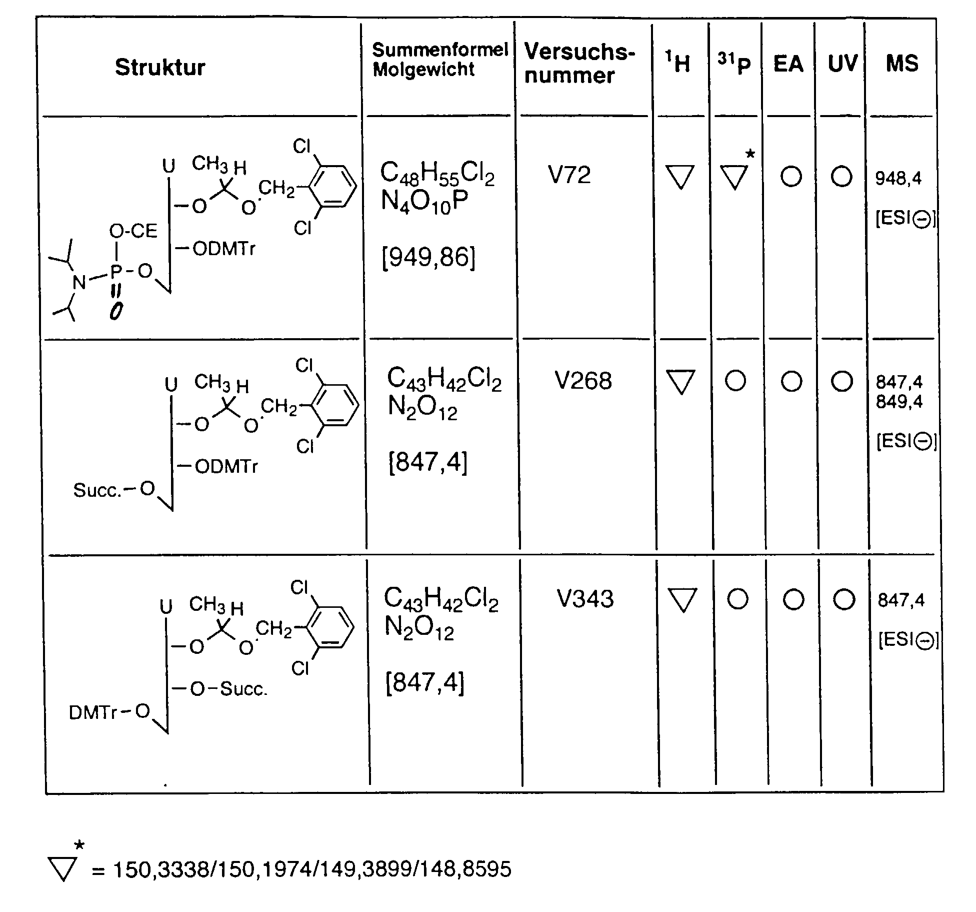

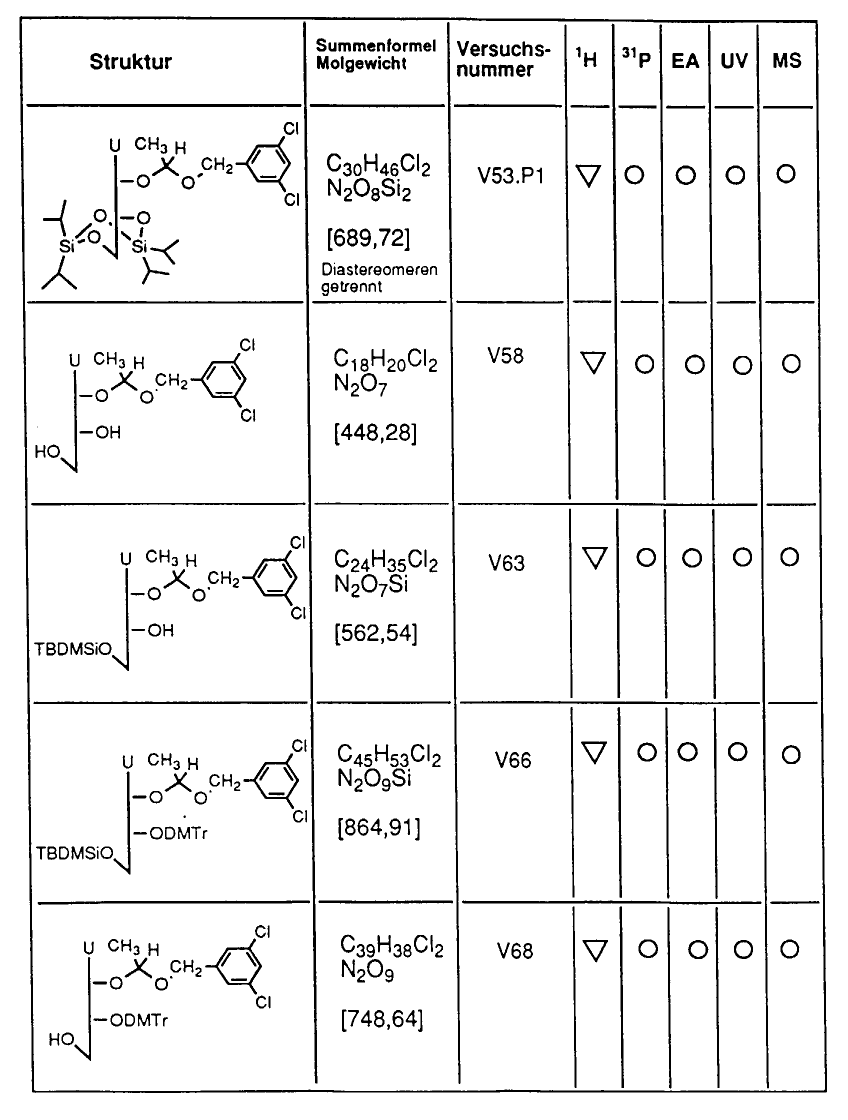

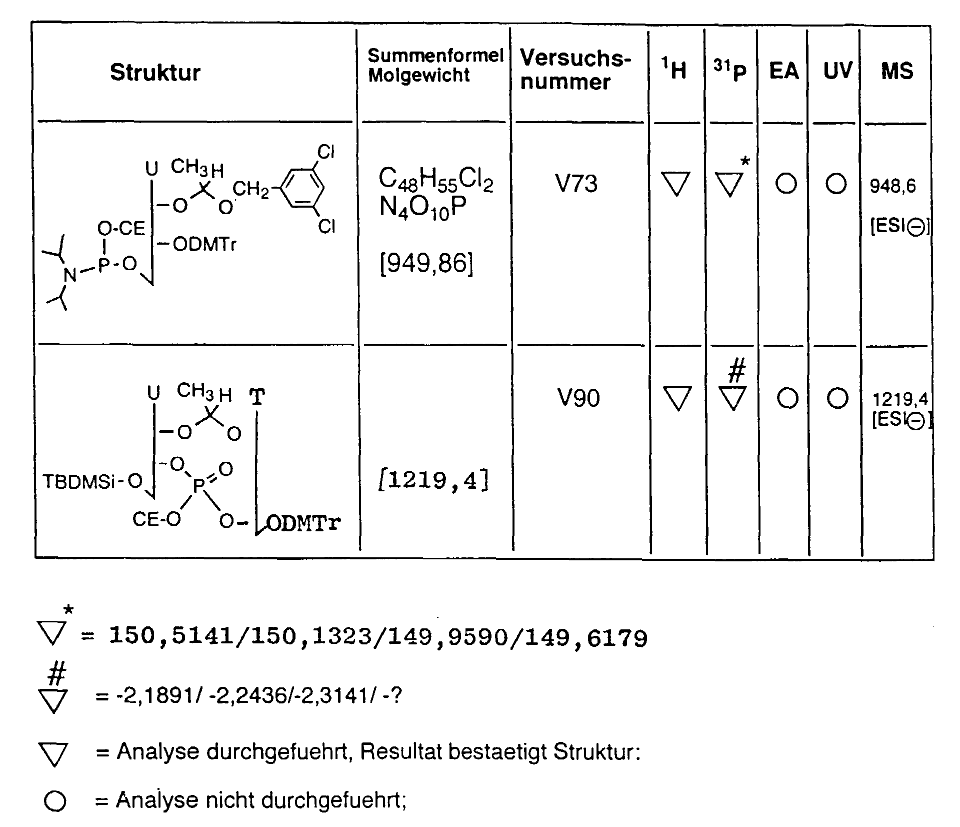

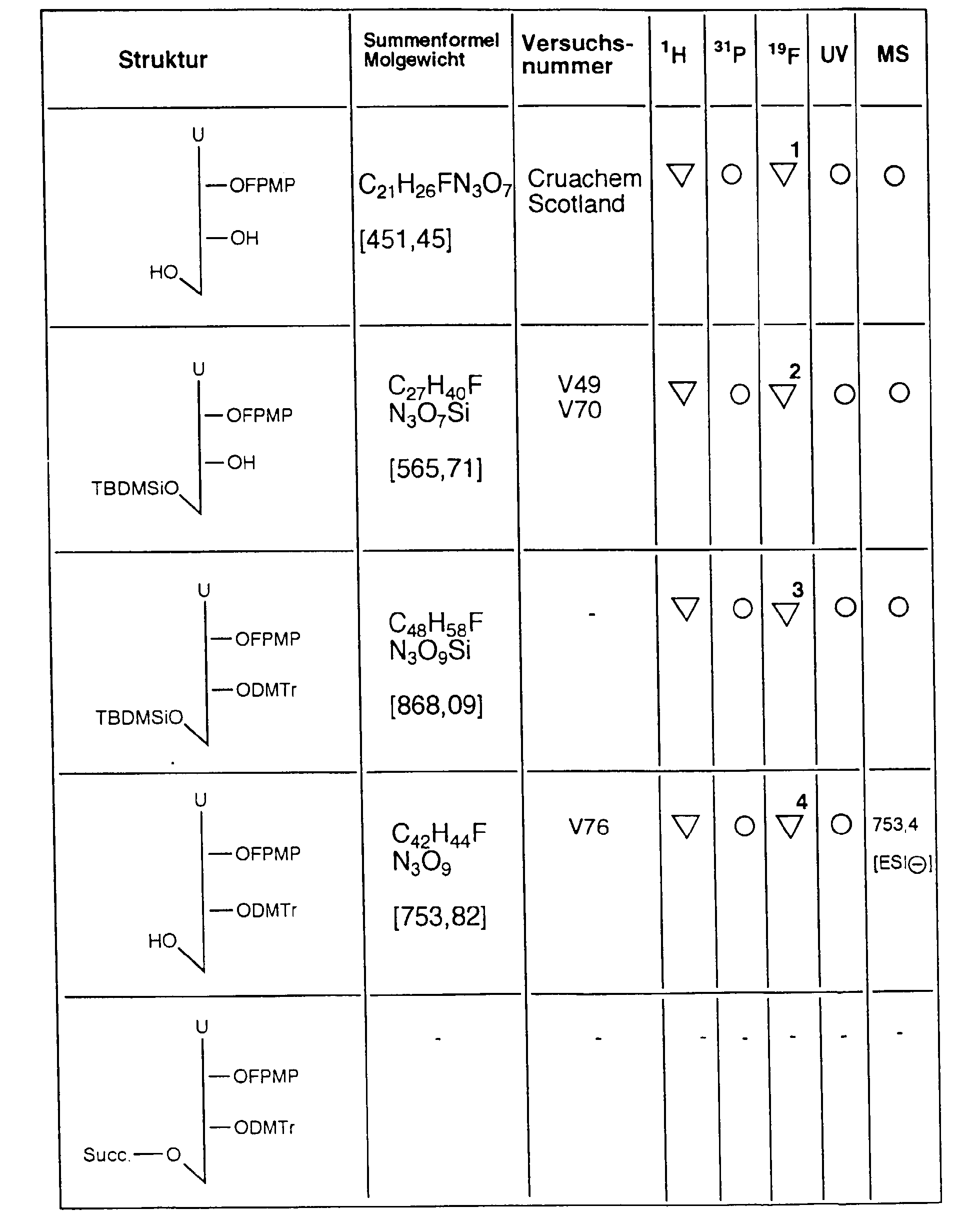

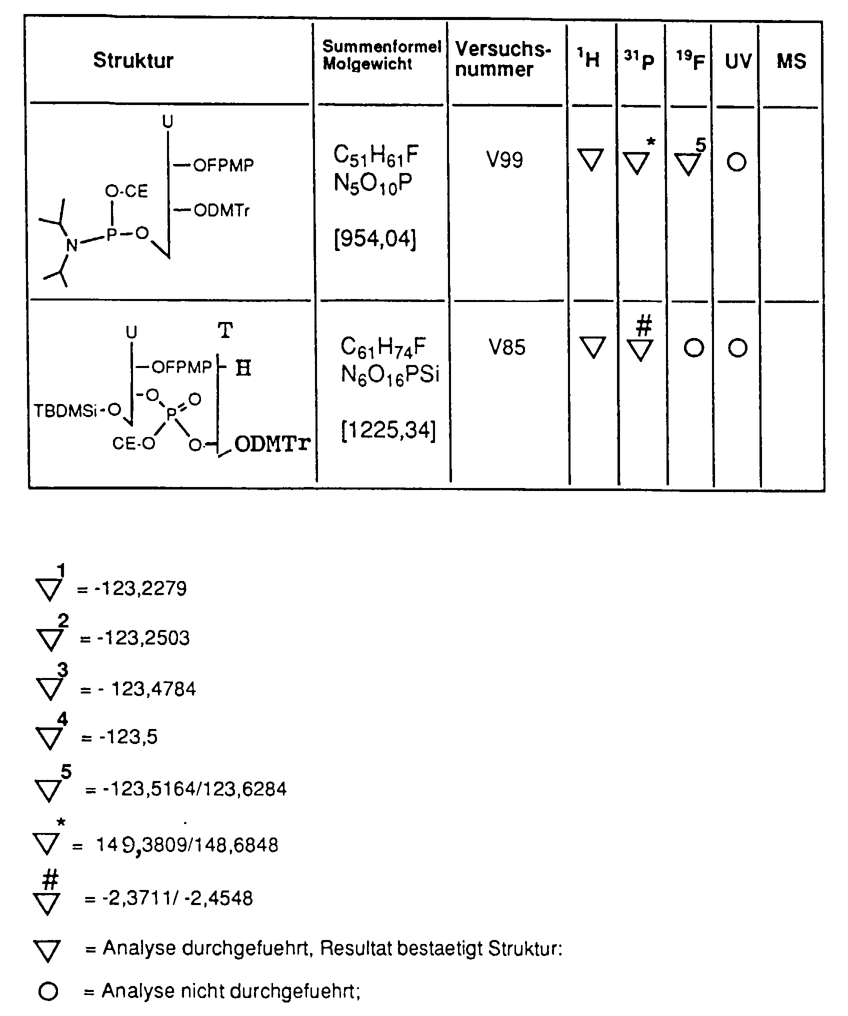

### F) Beispiele für poröse Frittenböden

Reaktionsschema:

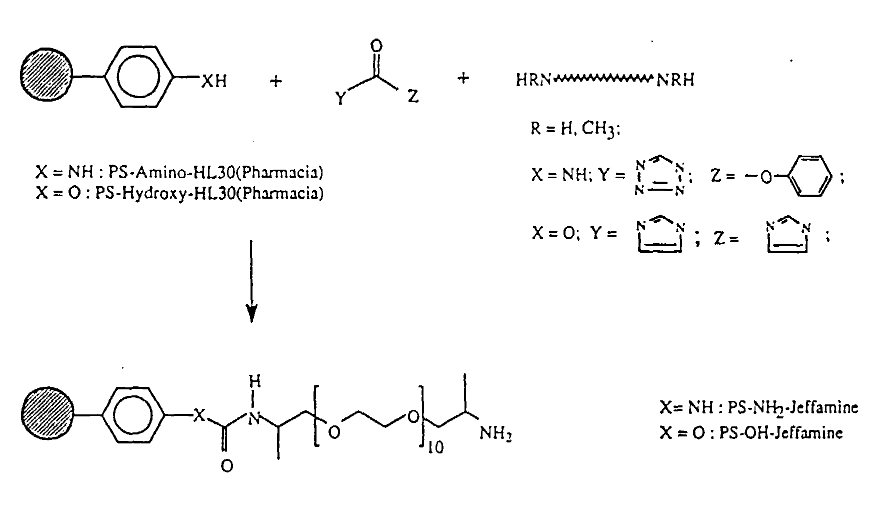

| **Name** | **-X** | **Trägermaterial** | **Versuchsnummer** | **Beladungsnummer** | **Capping** | **Beladungszahl** |
|---|---|---|---|---|---|---|
| PS-NH₂-Jeffamine | -NH | PS-Amino-HL30 | -- | V224A | -- | -- |
| Bedingungen: 106mg (15,2µmol)Träger; 18,5mg (97,9µmol) PCT | | | | | | |
| Aktivierung: 18h/RT/lml AcN); 1,75mmol Jeffamine 500; 24h/RT+5h/60°C | | | | | | |
| PS-NH₂-Jeffamine | -NH | PS-Amino-HL30 | -- | V232A | -- | -- |
| Bedingungen: 510mg (73µmol)Träger; 163mg (862µmol) PCT | | | | | | |
| Aktivierung: 17h/RT/5ml AcN; 0,525mmol Jeffamine 500; 2,5h/RT+5,5h/60°C | | | | | | |
| PS-NH₂-Jeffamine | -NH | PS-Amino-HL30 | -- | V288B3 | | BPB: 60µmol/g |
| Bedingungen: 1,024g (145µmol)Träger; 200mg (1,053mmol) | | | | | | |
| Aktivierung: 24h/RT/20ml AcN);400mg akt. Träger+300µl Jeffamine 500/24h/60°Cl | | | | | | |
| Capping: 10ml DMF; 3ml Pyridin; 0,5ml Ac₂O; 15min/RT; | | | | | | |
| PS-OH-Jeffamine | -O | PS-Hydroxy-HL30 | -- | V232B | -- | BPB: 72µmol/g |
| Bedingungen: 357mg Träger; 137mg CDI (844µmol) | | | | | | |
| Aktivierung: 17h/RT/5ml AcN; 525µmol Jeffamine 500; 2,5h RT+5,5h/60°C | | | | | | |

Vorteile des Spacermoleküls:
- Nach Aktivierung der Aminofunktion können die nicht umgesetzten Funktionen auf der Trägeroberfläche mit Essigsäureanhydrid gecappt werden. Mögliche nebenreaktionen durch vicinale Hydroxyfunktionen (intramolekularer Ringschluß und Abspaltung vom Träger während der Ammoniakbehandlung) werden dadurch vermieden.
- Die Beladung kann durch Anfärben der nicht abreagierten basischen Aminofunktionen über den Bromphenolblau(BPB)-Test nach Aktivierung, Capping bzw. Aminolyse ermittelt werden.
- Durch die Verwendung von bisfunktionellen Aminopolyglykolspacern (z.B. Jeffamin 500) wird die hydrophobe Trägeroberfläche hydrophiler. Dies kann sich positiv auf die Reaktionsausbeuten bei der Oligomerensynthese auswirken (z.B. verringert sich die elektrostatische Aufladung, die hydrophilen Spacerarme ragen weiter in polare Lösungsmittel hinaus etc.)

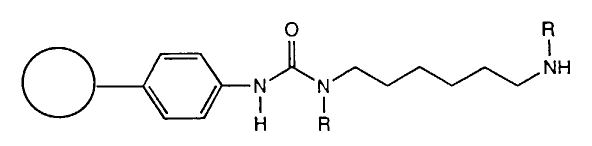

| **Name** | **-X (-R)** | **Trägermaterial** | **Versuchsnummer** | **Beladungsnummer** | **Capping** | **Beladungszahl** |
|---|---|---|---|---|---|---|
| PS-NH₂-Amino-hexane | -NH (-H) | PS-Amino-HL30 | -- | 277 | | BPB: 102µmol/g |
| Bedingungen: 500mg (71,5µmol)Träger; 50mg PCT (264µmol); 24h/RT;600µmol 1,6-Diaminohexane 4,0ml AcN/DMF(1/1) 60°C/12h | | | | | | |
| Capping: Ac₂O DMF Pyridin (0,5/5/2ml) 500mg DMAP 10 min/RT | | | | | | |
| PS-NH₂-Amino-hexane | -NH (-H) | PS-Amino-HL30 | -- | V288.B4 | -- | BPB: 188µmol/g (?) |
| Bedingungen: 1,024g (144µmol)Träger; 200mg PCI (1053µmol); 24h/RT; 600 mg alkt. Träger+420mg 1,6-Diaminohexane 12h/60°C | | | | | | |
| PS-NH₂-Methyl-amino-hexane | -NH | PS-Amino-HL30 | -- | V312 | | BPB: 97µmol/g |
| Bedingungen: 1,031g (145µmol)Träger; 125mg PCT (0,661mmol); | | | | | | |
| Aktivierung: 3h/RT/10ml AcN; 300mg N,N'-Dimethyl-1,6-hexanediamine; 16h/60°C | | | | | | |
| Capping: Ac₂O DMF Pyridin (0,5/5/2ml) 500mg DMAP 15 min/RT | | | | | | |

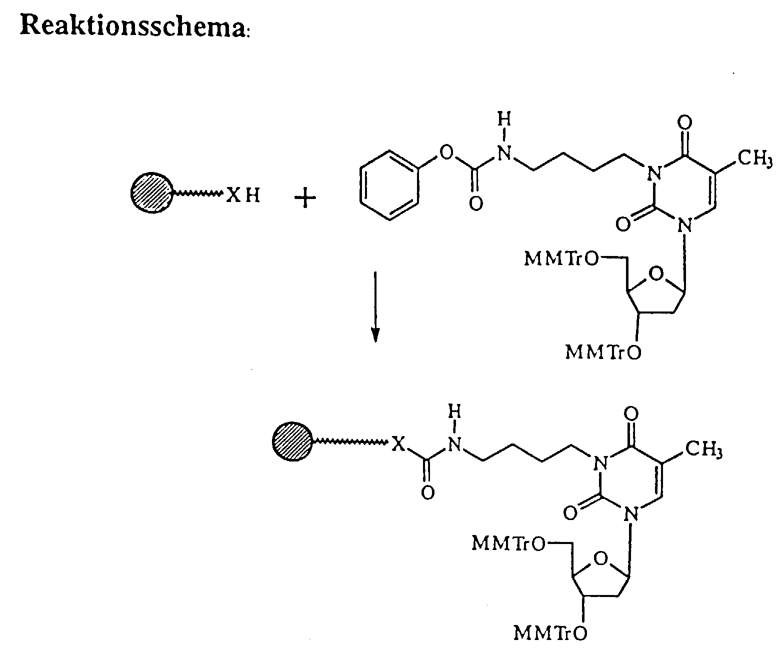

| **Name** | **-X** | **Trägermaterial** | **Versuchsnummer (Spacer)** | **Beladungsnummer** | **Capping** | **Beladungszahl** |
|---|---|---|---|---|---|---|
| PS-NH₂-Thymidin-3',5'-MMTr | NH | PS-Amino-HL30 | V210 | V225 | | MMTr: 27µmol/g |
| Bedingungen: 118mg (17µmol)Träger; 8,2mg (8,4µmol); 15h/60°C/1,5ml AcN | | | | | | |
| Capping: Ac₂O DMF Pyridin (0,1/1,0/0,5ml) 100mg DMAP 60 min/RT | | | | | | |
| PS-NH₂-Jeffamine-Thymidin-3',5'-MMTr | NH | PS-NH₂-Jeffamine | V210/V232A | V240A | -- | MMTr: 15µmol |
| Bedingungen: 86mg V232A 7,8mg (8,0µmol)V210; 25h/60°C/1,0ml AcN | | | | | | |
| PS-OH-Jeffamine-Thymidin-3',5'-MMTr | O | PS-OH-Jeffamine-Thymidin-3',5-MMTr | V210/V232B | V240B | -- | MMTr: 18µmol |
| Bedingungen: 60mg V232B 7,7mg (7,9µmol)V210; 25h/60°C/1,0ml AcN | | | | | | |

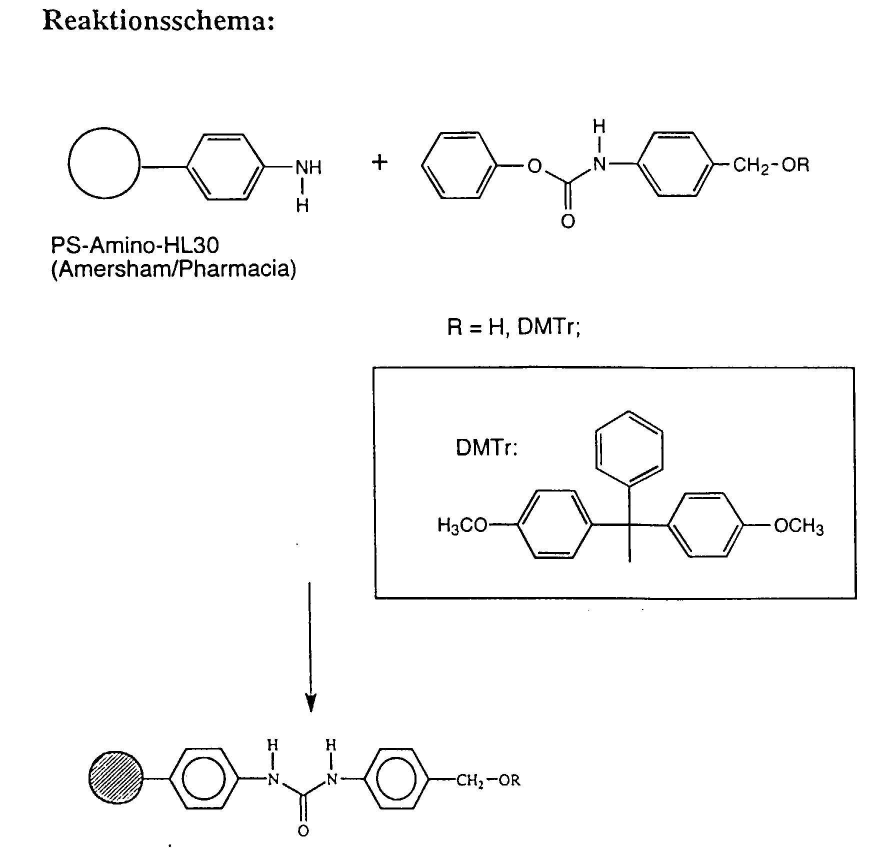

| Name | -R | Trägermaterial | Versuchsnummer | Beladungsnummer | Capping | Beladungszahl |
|---|---|---|---|---|---|---|
| PS-NH₂-Benzylalkohol-OH | -H | PS-Amino-HL30 | D744 | 310 | | Indirekt über BPB 110 µmol/g |
| Bedingungen: 500mg (71,5µmol)Träger; 80mg (329µmol=4,6 Moläquivalente) 20h/60-70°C in 5ml CH₃CN | | | | | | |
| Capping: DMAP Ac₂O CH₃CN 30min.RT | | | | | | |
| PS-NH₂-Benzylalkohol-ODMTr | -DMTr | PS-Amino-HL30 | V366-P1 | -- | | |
| Bedingungen: | | | | | | |

Vorteile des Spacermoleküls:
- Eine mögliche Nebenreaktion durch vicinale Hydroxyfunktionen wird vermieden.
- Die Beladung kann durch Anfärben der nicht abreagierten basischen Aminofunktionen über den Bromphenolblau(BPB)-Test ermittelt werden, insbesondere wenn R = H. Bei der tritylierten Variante kann die Beladung entweder über BPB oder nach dem Cappen über den Tritylwert bestimmt werden.
- Bei Beladung mit einem 3'-O-Phosphoramidit entstehen statt der säurelabilen Phosphorsäureamidate die stabileren Phosphorsäurediester (W. Bannwarth; Helv. Chim. Acta, Band 71, 1517 (1988). Dies ist im Hinblick auf die Verwendung von Safety-Catch-2'-O-Acetal-Linkern (Vergl. Anlage 5) von Vorteil!

Abbildung 1A: Molekulare Moldule für die Festphasensynthese von Oligonucleotiden. P = polymeres Trägermaterial; X = chemische Funktion zur Verankerung (-O- oder -NH-); Spacer = Abstandhalter; Linker = Einheit zur reversiblen Verankerung des 1. Nucleotidbausteins; N_{1,2}= Nucleotidbausteine
Abbildung 2: Reaktivität der Oberflächen verschiedener PE-Materialien im Tritylierungs-/Detritylierungstest; red. = reduktiv gesäubert
Abbildung 3: Gesteuerte Hydroxylierung von PE-Material
Abbildung 4: Beispiele für die in Abbildung 1 konzipierten modularen Syntheseträger. Ausgangsmaterial ist hydroxyliertes Polymer; 1 und 2 sind konventionelle Trägerbeladungen mit 3'-Nucleosidsuccinaten ohne und mit Spacer; 3 ist das Konzept des universellen Linkers (X = Acyl) und des "safety-catch"-Linkers (X = orthogonale Schutzgruppe), wobei das riboU-Nucleosid den Linker darstellt und nicht in das Oligonucleotid eingebaut wird; 4 ist ein Trägermaterial beladen für die Peptidsynthese
Abbildung 5: Beispiel für die Qualitäten von Oligonucleotid-Syntheseprodukten, hergestellt in einem konventionellen Synthesizer auf konventionellem Trägerharz (oben) und auf PE-Fritten-material derivatisiert nach Abb. 4, Typ 1

Sequenz: decameres Thymidylat ("Tr-off"), voll entschützt;
Automat: Phamacia Gene Assembler;
Trägermaterial: Phamacia T-Träger (CPG);
Linker: Succin
Synthese: optimiert

Sequenz: decameres Thymidylat (Tr-off"), voll entschützt;
Automat: Phamacia Gene Assembler;
Trägermaterial: funktionalisierte PE-Fritten (IRIS 45);
Linker: Succinat;
Synthese: nicht optimiert;

### Literatur

(2) Ronald Frank, Simultane kombinatorische Synthese: Moleküle nach Maß durch Screening aus der Vielfalt. In: GBF, Gesellschaft für Biotechnologische Forschung mbH Braunschweig, wissenschaftlicher Ergebnisbericht (1993), 5 - 16
(5B) Frank & Döring in Tetrahedron, 44 (1988), 6031 - 6040
(5C) Frank et al. in Nucleic Acids Res. 11 (1983), 4365 - 4377
(6) Bray et al. in Tetrahedron Letters, 40 (1990), 5811 - 5814
(7) Hoffmann & Frank in Tetrahedron Letters, 42 (1994), 7763 - 7766
(8) DE P 43 20 260.8; DE 44 31 317.9; PCT/EP 94/01896 (9A) EP 85 110 454.7 = 0 174 525
(14) Jung & Beck - Sickinger in Angew. Chem., 104 (1992), 375 - 500
(15) Frank in Bioorg. Med. Chem. Letters, 3 (1993), 425 - 430
(16) Lashkari et al. in PNAS, 92 (1995), 7912 - 7915
(17A) Moraj et al. in Biochem. J. 316 (1996), 193 - 199
(17B) Deibel et al. in Peptide Res., 2 (1989), 189 - 194

## Patentansprüche

1. Automatisiertes simultanes chemisches Syntheseverfahren, **dadurch gekennzeichnet,** daß für die kovalente Verknüpfung der Produkte mit Trägermaterial geeignete Verknüpfungsbausteine bzw. Linker vorgesehen werden, die eine selektive und schonende finale Abspaltung der Produkte erlauben, wobei insbesondere für die Synthese von oligomeren Verbindungen, insbesondere von Oligonucleotiden oder Peptiden, ein universeller Linker vorgesehen wird, an den auch die Bausteine der ersten Aufbaureaktion mit dem gleichen chemischen Reaktionstyp verknüpft werden können, der auch für die weiteren Aufbaureaktionen eingesetzt wird, wodurch für die ganze Synthese einer Verbindungsklasse nur ein Typ von Bausteinen gebraucht wird, insbesondere nur ein Nucleosid-3'-phosphoramidit für die Synthese von 3'-OH-Oligonucleotiden.

2. Verfahren nach Anspruch 1, **dadurch gekennzeichnet,** daß der Linker bei den finalen Abspaltungsreaktionen in an sich bekannter Weise erst in eine labile, aber noch intakte Form überführt wird, d. h. einen safety-catch-Linker, die dann durch eine milde chemische Behandlung, vorzugsweise eine pH-Veränderung, gespalten wird, wobei man insbesondere die kovalent fixierten Produkte einfach durch automatische Waschoperationen an Trägermaterial von chemischen Reagenzien reinigt und erst ganz zum Schluß aus den Reaktoren in eine der Reaktoranordnung komplementäre Anordnung von Auffanggefäßen eluiert (Fig. 9).

3. Verfahren nach Anspruch 1 oder 2, **dadurch gekennzeichnet,** daß die Zielprodukte der Synthesen mit einer als Affinitätslabel nutzbaren Gruppe versehen werden, über die die Zielprodukte an eine entsprechende Affinitätsphase gebunden werden können, wobei man insbesondere die aus den Reaktoren eluierten Produkte in eine der Reaktoranordnung komplementäre Anordnung von Affinitätssäulen (Fig. 9) überführt und durch automatisierte Waschoperationen von Nebenprodukten reinigt, wonach man die Zielprodukte durch automatisierte Wasch- oder Abspaltungsoperationen aus den Affinitätssäulen in eine der Reaktoranordnung komplementäre Anordnung von Auffanggefäßen eluiert.

4. Verfahren nach Anspruch 1, 2 oder 3, wobei man Affinitätssäulen mit Bindungskapazitäten verwendet, die derart limitiert sind, daß auch bei unterschiedlichen Syntheseausbeuten je Reaktor eine gleiche Mindestmenge an Zielprodukt gebunden und eluiert wird, so daß alle Produkte einer multiplen Synthese in äquimolaren Mengen erhalten werden können.
